# EUROPEAN PATENT SPECIFICATION

(11) **EP 1 037 918 B1**
(45) Date of publication and mention of the grant of the patent: **04.03.2009**
(21) Application number: 98963052.0
(22) Date of filing: 11.12.1998
(51) Int. Cl.: A61K 39/39, C07K 14/52, A61K 38/19, A61K 48/00, A61K 39/21

(54) **COMPOSITIONS TO ENHANCE THE EFFICACY OF A VACCINE USING CHEMOKINES**
ZUSAMMENSETZUNGEN ZUR STEIGERUNG DER EFFIZIENZ VON IMPFSTOFFEN UNTER VERWENDUNG VON CHEMOKINEN
COMPOSITIONS POUR AMELIORER L'EFFICACITE D'UN VACCIN UTILISANT DES CHEMOKINES

(30) Priority: 11.12.1997 US 69281 P
(43) Date of publication of application: 27.09.2000
(73) Proprietor: UNIVERSITY OF MARYLAND BIOTECHNOLOGY INSTITUTE, College Park, MD 20740 (US)
(72) Inventor: GALLO, Robert, C., Bethesda, MD 02817 (US); DEVICO, Anthony, L., Alexandria, VA 22304 (US); GARZINO-DEMO, Alfredo, Baltimore, MD 21201 (US)
(74) Representative: Wright, Robert Gordon McRae
(86) International application number: PCT/US1998/026291
(87) International publication number: WO 1999/029728

(56) References cited:
- WO-A-96/40267
- WO-A-96/40923
- US-A- 5 141 867
- PAL RANAJIT ET AL: "Inhibition of HIV-1 infection by the beta-chemokine MDC" SCIENCE (WASHINGTON D C), vol. 278, no. 5338, 24 October 1997 (1997-10-24), pages 695-698, XP002097214 ISSN: 0036-8075
- FURCI LUCINDA ET AL: "Antigen-driven C-C chemokine-mediated HIV-1 suppression by CD4+ T cells from exposed uninfected individuals expressing the wild-type CCR-5 allele" JOURNAL OF EXPERIMENTAL MEDICINE, vol. 186, no. 3, 1997, pages 455-460, XP002295797 ISSN: 0022-1007
- ENG et al., "The Stimulatory Effects of Interleukin (IL)-12 on Hematopoiesis are Antagonized by IL-12-Induced Inferferon gamma In Vivo", J. EXP. MED., May 1995, Vol. 181, pages 1893-1898, XP002918730
- ORANGE et al., "Mechanism of Interleukin 12-Mediated Toxicities During Experimental Viral Infections: Role of Tumor Necrosis Factor and Glucocorticoids", J. EXP. MED., March 1995, Vol. 181, pages 901-914, XP002918731
- WU et al., "Receptor-Mediated In Vitro Gene Transformation by a Soluble DNA Carrier System", THE JOURNAL OF BIOLOGICAL CHEMISTRY, 05 April 1987, Vol. 252, No. 10, pages 4429-4432, XP002918732
- Kim, J.J. et al (1997) J. Immunol. 158:816-826

## Description

### 1. CROSS-REFERENCE TO RELATED APPLICATIONS

This application claims priority to U.S. Application Serial No. 60/069,281 filed December 11, 1997.

### 2. BACKGROUND OF THE INVENTION

The present invention relates to compositions useful for enhancing the efficacy of a vaccine by administration of a chemokine, such as macrophage derived chemokine (MDC), in conjunction with the vaccine.

### 2.1. GENERATION OF AN IMMUNE RESPONSE

The introduction of a foreign antigen into an individual elicits an immune response consisting of two major components, the cellular and humoral immune responses, mediated by two functionally distinct populations of lymphocytes known as T and B cells, respectively (*see generally* Coutinho, 1991, Immune System, Encyclopedia of Human Biology, Vol. 4, Ed. Dulbecco, Academic Press, Inc.). A subset of T cells responds to antigen stimulation by producing lymphokines which "help" or activate various other cell types in the immune system.

Another T cell subset is capable of developing into antigen-specific cytotoxic effector cells, which can directly kill antigen-positive target cells. On the other hand, the B cell response is primarily carried out by secretory protein, antibodies, which directly bind and neutralize antigens.

Helper T cells (TH) can be distinguished from classical cytotoxic T lymphocytes (CTL) and B cells by their cell surface expression of the glycoprotein marker CD4. Although the mechanism by which CD4⁺ TH function has not been fully elucidated, the existence of functionally distinct subsets within the CD4⁺ T cell compartment has been reported (Mosmann and Coffman, 1989, Ann. Rev. Immunol. 7:145-173). In the mouse, type 1 helper T cells (TH1) produce interleukin-2 (IL-2) and τ-interferon (τ-IFN) upon activation, while type 2 helper T cells (TH2) produce IL-4 and IL-5. Based on the profile of lymphokine production, TH1 appear to be involved in promoting the activation and proliferation of other T cell subsets including CTL, whereas TH2 specifically regulate B cell proliferation and differentiation, antibody synthesis, and antibody class switching.

A second T cell subpopulation is the classical CTL which express the CD8 surface marker. Unlike most TH, these cells display cytolytic activity upon direct contact with target cells, rather than through the production of lymphokines. *In vivo*, CTL function is particularly important in situations where an antibody response alone is inadequate. Significant experimental evidence indicates that CTL rather than B cells and their antibody products play a principal role in the defense against viral infections and cancer.

A salient feature of both T and B cell responses is their exquisite specificity for the immunizing antigen; however, the mechanisms for antigen recognition differ between these two cell types. B cells recognize antigens by antibodies, either acting as cell surface receptors or as secreted proteins, which bind directly to antigens on a solid surface or in solution, whereas T cells only recognize antigens that have been processed or degraded into small fragments and presented on a solid phase such as the surface of antigen-presenting cells (APC). Additionally, antigenic fragments must be presented to T cells in association with major histocompatibility complex (MHC)-encoded class I or class II molecules. The MHC refers to a cluster of genes that encode proteins with diverse immunological functions. In man, the MHC is known as HLA. Class I gene products are found on all somatic cells, and they were originally discovered as targets of major transplantation rejection responses. Class 11 gene products are mostly expressed on cells of various hematopoietic lineages, and they are involved in cell-cell interactions in the immune system. Most importantly, MHC-encoded proteins have been shown to function as receptors for processed antigenic fragments on the surface of APC (Bjorkman et al., 1987, Nature 329:506-512).

Another level of complexity in the interaction between a T cell and an antigenic fragment is that it occurs only if the MHC molecules involved are the same on the APC and the responding T cells. In other words, a T cell specific for a particular antigenic epitope expresses a receptor having low affinity for self MHC proteins, which when such MHC proteins on APC are occupied by the epitope, engage the T cell in a stronger interaction leading to antigen-specific T cell activation. The phenomenon of a T cell reacting with a processed antigen only when presented by cells expressing a matching MHC is known as MHC-restriction.

The specificity of T cell immune responses for antigens is a function of the unique receptors expressed by these cells. The T cell receptor (TCR) is structurally homologous to an antibody; it is a heterodimer composed of disulfide-linked glycoproteins. Four TCR polypeptide chains known as α, β, τ, and δ have been identified, although the vast majority of functional T cells express the αβ heterodimeric TCR. Transfer of α and β genes alone into recipient cells was shown to be both necessary and sufficient to confer antigen specificity and MHC-restriction (Dembic et al., 1986, Nature 320:232-238). Thus, the αβ TCR appears to be responsible for recognizing a combination of antigenic fragment and MHC determinants.

The apparent basis of MHC restriction is that CD4⁺ T cells express αβ TCR which recognize antigenic fragments physically associated with MHC class II proteins, while the TCR on CD8⁺ CTL recognize MHC class I-associated fragments. Thus, CD4⁺ T cells can recognize only a restricted class of APC that are class II⁺, whereas CD8⁺ CTL can interact with virtually any antigen-positive cells, since all cells express class I molecules. CD4⁺ CTL have been identified, and they are MHC class II restricted, and lyse target cells only if the latter express self-MHC class II determinants associated with specific antigenic fragments. Both CD4 and CD8 molecules also contribute to this interaction by binding to monotypic determinants on the MHC class II and I molecules, respectively.

A second type of TCR composed of τδ heterodimers is expressed by a small percentage of T cells, but the involvement of τδ T cells in antigen-specific recognition is still poorly understood. Some studies have shown that functionally active τδ T cells can be cytolytic in a MHC non-restricted manner.

In summary, the generation of an immune response begins with the sensitization of CD4⁺ and CD8⁺ T cell subsets through their interaction with APC that express MHC-class I or class II molecules associated with antigenic fragments. The sensitized or primed CD4⁺ T cells produce lymphokines that participate in the activation of B cells as well as various T cell subsets. The sensitized CD8⁺ T cells increase in numbers in response to lymphokines and are capable of destroying any cells that express the specific antigenic fragments associated with matching MHC-encoded class I molecules. For example, in the course of a viral infection, CTL eradicate virally-infected cells, thereby limiting the progression of virus spread and disease development.

### 2.2. ANTIGEN PRESENTING CELLS

The presentation of antigens to T cells is carried out by specialized cell populations referred to as antigen presenting cells (APC). Typically, APC include macrophages/monocytes, B cells, and bone marrow derived dendritic cells (DC). APC are capable of internalizing exogenous antigens, cleaving them into smaller fragments in enzyme-rich vesicles, and coupling the fragments to MHC-encoded products for expression on the cell surface (Goldberg and Rock, 1992, Nature 357:375-379). Since APC express both MHC-encoded class I and class II glycoproteins, they can present antigenic fragments to both CD4⁺ and CD8⁺ T cells for the initiation of an immune response.

By definition, APC not only can present antigens to T cells with antigen-specific receptors, but can provide all the signals necessary for T cell activation. Such signals are incompletely defined, but probably involve a variety of cell surface molecules as well as cytokines or growth factors. Further, the factors necessary for the activation of naive or unprimed T cells may be different from those required for the re-activation of previously primed memory T cells. The ability of APC to both present antigens and deliver signals for T cell activation is commonly referred to as an accessory cell function. Although monocytes and B cells have been shown to be competent APC, their antigen presenting capacities *in vitro* appear to be limited to the re-activation of previously sensitized T cells. Hence, they are not capable of directly activating functionally naive or unprimed T cell populations.

Although it had been known for a long time that APC process and present antigens to T cells, it was not shown until relatively recently that small antigenic peptides could directly bind to MHC-encoded molecules (Babbit et al., 1985, Nature 317:359; Townsend et al., 1986, Cell 44:959). However, it is believed that, normally, complex antigens are proteolytically processed into fragments inside the APC, and become physically associated with the MHC-encoded proteins intracellularly prior to trafficking to the cell surface as complexes. Two distinct pathways for antigen presentation have been proposed (Braciale et al., 1987, Immunol. Rev. 98:95-114). It was thought that exogenous antigens were taken up by APC, processed and presented by the exogenous pathway to class II restricted CD4⁺ T cells, while the endogenous pathway processed intracellularly synthesized proteins, such as products of viral genes in virally-infected cells, for association with MHC class I proteins and presentation to CD8⁺ CTL. However, although the two pathways in antigen processing and presentation may still be correct in some respects, the distinction is blurred in light of recent findings that exogenously added antigens may also be presented to class I-restricted CTL (Moore et al., 1988, Cell 54:777).

The term "dendritic cells" (DC) refers to a diverse population of morphologically similar cell types found in a variety of lymphoid and non-lymphoid tissues (Steinman, 1991, Ann. Rev. Immunol. 9:271-296). These cells include lymphoid DC of the spleen, Langerhans cells of the epidermis, and veiled cells in the blood circulation. Although they are collectively classified as a group based on their morphology, high levels of surface MHC-class II expression, and absence of certain other surface markers expressed on T cells, B cells, monocytes, and natural killer cells, it is presently not known whether they derive from a common precursor or can all function as APC in the same manner. Further, since the vast majority of published reports have utilized DC isolated from the mouse spleen, results from these studies may not necessarily correlate with the function of DC obtained from other tissue types. (Inaba et al., 1997, J. Exp. Med. 166:182-194; Hengel et al., 1987, J. Immunol., 139:4196-4202; Kaut et al., 1988, J. Immunol., 140:3186-3193; Romani et al., 1989, 1. Exp. Med. 169:1169-1178; Macatonia et al., 1989, J. Exp. Med. 169:1255-1264; Inaba et al., 1990, J. Exp. Med. 172:631-6640). For example, despite high levels of MHC-class II expression, mouse epidermal Langerhans cells, unlike splenic DC, are not active APC in mixed leucocyte reaction (MLR), unless cultured with granulocyte-macrophage colony stimulating factor (GM-CSF) (Witmer-Pock et al., 1987, J. Exp. Med. 166:1484-1498; Heufler et al., 1988, J. Exp. Med. 167:700-705). Most human Langerhans cells express the CD1 and CD4 markers, while blood DC do not. Additionally, it has not been established the extent to which the functional characteristics observed with mouse DC are applicable to human DC, especially the DC obtained from non-splenic tissues; in part, due to inherent differences between the human and murine immune systems.

Recently, a few studies have described the isolation of human DC from the peripheral blood, which involves the use of sheep red blood cells and/or fetal calf serum (Young and Steinman, 1990, J. Exp. Med. 171 :1315-1332; Freudenthal and Steinman, 1990, Proc. Natl. Acad. Sci. USA 87:7698-7702; Macatonia et al., 1989 Immunol. 67:285-289; Markowicz and Engleman, 1990, J. Clin. Invest. 85:955-961). Engleman et al. described a partial purification procedure of DC from human blood, which does not involve the use of sheep red blood cells and/or fetal calf serum, and showed that the partially purified human DC can, in fact, present exogenous antigens to naive T cells (PCT Publication WO 94/02156 dated February 3, 1994 at page 9, lines 5-32).

Recent studies have indicated that DCs are superior APCs as compared to other APCs such as macrophages and monocytes. First, the potent accessory cell function of DCs provides for an antigen presentation system for virtually any antigenic epitopes which T and B cells are capable of recognizing through their specific receptors. For example, Engleman et al. demonstrate that human DCs can present both complex protein antigens and small peptides to CD4⁺ T cells as well as to as CD8⁺ CTL (PCT Publication WO 94/02156 dated February 3, 1994, Example 7, from page 29, line 10 to page 34, line 16). Engleman et al. also show that the *in vitro* priming effect of DCs does not require the addition of exogenous lymphokines, indicating that DCs produce all of the necessary signals in antigen presentation leading to the activation of T cells (PCT Publication WO 94/02156 dated February 3, 1994, from page 32, line 36 to page 33, line 2). More importantly, DCs can induce a primary CD4⁺ T cell-mediated proliferative response when similarly prepared monocytes can not induce such a response (PCT Publication WO 94/02156 dated February 3, 1994 at page 31, lines 23-30). Similarly, when DCs and monocytes ware compared for their ability to present antigens for re-activating secondary T cell response, it was observed that DCs were capable of stimulating a stronger response than monocytes (PCT Publication WO 94/02156 dated February 3, 1994 at page 32, lines 12-16).

### 2.3. CHEMOKINES

Chemokines, or chemoattractant cytokines, are a subgroup of immune factors that have been shown to mediate chemotactic and other pro-inflammatory phenomena (see, Schall, 1991, Cytokine 3:165-183). Chemokines are small molecules of approximately 70-80 residues in length and can generally be divided into two subgroups, α which have two N-terminal cysteines separated by a single amino acid (CxC) and 13 which have two adjacent cysteines at the N terminus (CC). RANTES, MIP-1 a and MIP- 10 are members of the β subgroup (reviewed by Horuk, R., 1994, Trends Pharmacol. Sci. 15:159-165; Murphy, P.M., 1994, Annu. Rev. Immunol. 12:593-633; Baggiolini et al. Annu. Rev. Immuno/. 1997, 15:675-705).

MCP-1 has been shown to attract monocytes but not neutrophils. MCP-1, MCP-2, and MCP-3 share a pyroglutamate proline NH₂-terminal motif and are structurally closely related to each other and to eotaxin (56% to 71% amino acid sequence identity). MCP-1, MCP-2, and MCP-3 attract monocytes, CD4⁺ and CD8⁺ T lymphocytes (Loetscher et al. FAESB J. 1994, 8:1055-60), as well as basophil leukocytes. MCP-2, MCP-3, and MCP-4 (but not MCP-1) attracts eosinophil leukocytes. All four MCPs attract activated T lymphocytes, natural killer (NK) cells, and dendritic cells (see Baggiolini et al. Annu. Rev. Immunol. 1997, 15:675-705).

Eotaxin acts on eosinophils and is inactive on neutrophils and monocytes, but has weak-to-moderate chemotactic activity toward IL-2-conditioned T lymphocytes (see Baggiolini et al. Annu. Rev. Immunol. 1997, 15:675-705). Due to its preferential, powerful action on eosinophils and its occurrence in different species, eotaxin is considered to be an important chemokine in the pathophysiology of allergic conditions and asthma (See Baggiolini et al. Annu. Rev. Immunol. 1997, 15:675-705).

IP10 is a CXC chemokine attracts human monocytes, T lymphocytes, and NK cells, and Mig attracts tumor-infiltrating T lymphocytes. It has been suggested that IP10 and Mig may also be involved in the regulation of lymphocyte recruitment and the formation of the lymphoid infiltrates observed in autoimmune inflammatory lesions, delayed-type hypersensitivity, some viral infections, and certain tumors (Baggiolini et al. Annu. Rev. Immunol. 1997, 15:675-705).

SDF-1 (stromal cell-derived factor 1), including SDF-1 and SDF-1β stimulates the proliferation of B cell progenitors, and attracts mature dendritic cells (Finkel et al. Immunobiology 1998, 198:490-500). Synthetic human SDF-1 stimulates monocytes, neutrophils, and peripheral blood lymphocytes, as is indicated by [Ca2+]i changes and chemotaxis. SDF-1 is also a powerful HIV-suppressive factor (See Baggiolini et al. Annu. Rev. Immunol. 1997, 15:675-705).

The amino terminus of the β chemokines RANTES, MCP-1, and MCP-3 has been implicated in the mediation of cell migration and inflammation induced by these chemokines. This involvement is suggested by the observation that the deletion of the amino terminal 8 residues of MCP-1, amino terminal 9 residues of MCP-3, and amino terminal 8 residues of RANTES and the addition of a methionine to the amino terminus of RANTES, antagonize the chemotaxis, calcium mobilization and/or enzyme release stimulated by their native counterparts (Gong et al., 1996, J. Biol. Chem. 271:10521-10527; Proudfoot et al., 1996 J. Biol. Chem. 271:2599-2603). Additionally, α chemokine-like chemotactic activity has been introduced into MCP-1 via a double mutation of Tyr 28 and Arg 30 to leucine and valine, respectively, indicating that internal regions of this protein also play a role in regulating chemotactic activity (Beall et al., 1992, J. Biol. Chem. 267:3455-3459).

The monomeric forms of all chemokines characterized thus far share significant structural homology, although the quaternary structures of α and β groups are distinct. While the monomeric structures of the β and α chemokines are very similar, the dimeric structures of the two groups are completely different. An additional chemokine, lymphotactin, which has only one N terminal cysteine has also been identified and may represent an additional subgroup (γ) of chemokines (Yoshida et al., 1995, FEBS Lett. 360:155-159; and Kelner et al., 1994, Science 266:1395-1399).

Receptors for chemokines belong to the large family of G-protein coupled, 7 transmembrane domain receptors (GCR's) (See, reviews by Horuk, R., 1994, Trends Pharmacol. Sci. 15:159-165; and Murphy, P.M., 1994, Annu. Rev. Immunol. 12:593-633). Competition binding and cross-desensitization studies have shown that chemokine receptors exhibit considerable promiscuity in ligand binding. Examples demonstrating the promiscuity among β chemokine receptors include: CCR-1, which binds RANTES and MIP-1α (Neote et al., 1993, Cell 72:415-425), CCR-4, which binds RANTES, MIP-1α, and MCP-1 (Power et al., 1995, J. Biol. Chem. 270:19495-19500), and CCR-5, which binds RANTES, MIP-1α, and MIP-1β (Alkhatib et al., 1996, Science 272:1955-1958 and Dragic et al., 1996, Nature 381:667-674). Erythrocytes possess a receptor (known as the Duffy antigen) which binds both α and β chemokines (Horuk et al., 1994, J. Biol. Chem. 269:17730-17733; Neote et al., 1994, Blood 84:44-52; and Neote et al., 1993, J. Biol. Chem. 268:12247-12249). Thus the sequence and structural homologies evident among chemokines and their receptors allow some overlap in receptor-ligand interactions.

Godiska et al. identified and described the nucleic acid and amino acid sequences of an additional β chemokine designated macrophage derived chemokine (MDC) (PCT Publication WO 96/40923 dated December 19, 1996, and 1997, J. Exp. Med. 185:1595-1604). PCT publication WO 96/40923 further provides materials and methods for the recombinant production of the chemokine, the purified and isolated chemokine protein, and polypeptide analogues thereof. The PCT publication WO 96/40923 does not disclose that the human MDC has chemotactic activity upon DC. While Godiska et al. (1997, J. Exp. Med. 185:1595-1604) showed that, in a microchamber migration assay, monocyte-derived DC migrated toward the human MDC, the reference fails to teach that MDC can enhance an immune response to an antigen *in vivo.*

Chang et al. (1997, J. Biol. Chem. 272(40):25229-25237), isolated a stimulated T cell chemotactic protein (STCP-1) from an activated macrophage cDNA library. The nucleotide sequence of the STCP-1 is identical to that of the MDC isolated by Godiska et al. (PCT Publication WO 96/40923 dated December 19, 1996, and 1997, J. Exp. Med. 185:1595-1604). However, unlike the results observed by Godiska et al. (1997, J. Exp. Med. 185:1595-1604), Chang et al. (1997, J. Biol. Chem. 272(40):25229-25237) showed that although the STCP-1 acted as a mild chemoattractant for primary activated T lymphocytes and a potent chemoattractant for chronically activated T lymphocytes, the STCP-1 has no chemoattractant activity for monocytes, neutrophils, eosinophils and resting T lymphocytes. Chang et al. further showed that the STCP-1 does not induce Ca2⁺ mobilization in monocytes, dendritic cells, neutrophils, eosinophils, lipopolysaccharide-activated B lymphocytes, and freshly isolated resting T lymphocytes.

### 2.4. HIV VACCINES

Human immunodeficiency virus (HIV) induces a persistent and progressive infection leading, in the vast majority of cases, to the development of the acquired immunodeficiency syndrome (AIDS) (Barre-Sinoussi et al., 1983, Science 220:868-870; Gallo et al., 1984, Science 224:500-503). The HIV envelope surface glycoproteins are synthesized as a single 160 kilodalton precursor protein which is cleaved by a cellular protease during viral budding into two glycoproteins, gp41 and gp120. gp41 is a transmembrane glycoprotein and gp120 is an extracellular glycoprotein which remains non-covalently associated with gp41, possibly in a trimeric or multimeric form (Hammerskjold, M. and Rekosh, D., 1989, Biochem. Biophys. Acta 989:269-280). The V3 loop of gp120 is the major determinant of sensitivity to chemokine inhibition of infection or replication (Cocchi et al., 1996, Nature Medicine 2:1244-1247; and Oravecz et al., 1996, J. Immunol. 157:1329-1332).

Although considerable effort is being put into the design of effective therapeutics, currently no curative anti-retroviral drugs against AIDS exist. The HIV-1 envelope proteins (gp160, gp120, gp41) have been shown to be the major antigens for neutralizing anti-HIV antibodies present in AIDS patients (Barin et al., 1985, Science 228:1094-1096). Thus far, therefore, these proteins seem to be the most promising candidates to act as antigens for anti-HIV vaccine development. Several groups have begun to use various portions of gp160, gp120, and/or gp41 as immunogenic targets for the host immune system (see, for example, Ivanoff et al., U.S. Pat. No. 5,141,867; Saith et al., PCT publication WO 92/22654; Shafferman, A., PCT publication WO 91/09872; Formoso et al.; PCT publication WO 90/07119). Therefore, methods to increase the efficacy of vaccines against HIV, especially vaccines using gp120 as the antigen, are needed.

Additionally a novel vaccine technology, designated genetic vaccination, nucleic acid vaccination or DNA vaccination, has been explored to induce immune responses *in vivo*. Injection of cDNA expression cassettes results in *in* v*ivo* expression of the encoded proteins (Dubensky et al., 1984, Proc. Natl. Acad. Sci. USA 81:7529-7533; Raz et al., 1993, Proc. Natl. Acad. Sci. USA 90:4523; Wolff et al., 1990, Science 247:1465-1468), with the concomitant development of specific cellular and humoral immune responses directed against the encoded antigen(s) (Wang et al., 1995, Hum. Gene Ther. 6:407-418; Ulmer et al., 1993, Science 259:1745-1749; Tang et al., 1992, Nature 356:152-154; Michel et al., 1995, Proc. Natl. Acad. Sci. USA 92:5307-5311; and Lowrie et al., 1994, Vaccine 12:1537-1540). Humoral and cellular responses have been induced to HIV-1 and SIV antigens through various applications of this technology in macaques (Wang et al., 1995, Virology 221:102-112; Wang et al., 1993, Proc. Natl. Acad. Sci. USA 90:4156-4160; and Boyer et al., 1996, J. Med. Primatol. 25:242-250) as well as mice (Wang et al., 1995, Virology 221:102-1 12; Lu et al., 1995, Virology 209:147-154; Haynes et al., 1994, AIDS Res. Hum. Retroviruses 10 (Suppl. 2):543-545; Okuda et al., 1995, AIDS Res. Hum. Retroviruses 11:933-943).

Recently, Lekutis et al. (1997, J. lmmunol. 158:4471-4477), assessed the TH cell response elicited by an HIV-1 gp120 DNA vaccine in rhesus monkeys by isolation of gp120-specific, MHC class II-restricted CD4⁺ T cell lines from the vaccinated animals. Lekutis et al. showed that the isolated cell lines proliferated in response to APC in the presence of recombinant gp120, as well as to APC expressing HIV encoded env protein. Lekutis et al. further showed that these cell lines responded to env by secreting IFN-Γ and IFN-α without appreciable IL-4 production. These results demonstrate that the animals exhibited a cellular immune response to the DNA vaccine.

Boyer et al. (1997, Nature Medicine 3:625-532), inoculated chimpanzees with an HIV-1 DNA vaccine encoding env; rev, and gag/pol, and found that the immunized animals developed specific cellular and humoral immune responses to these proteins. After challenging the immunized animals with a heterologous chimpanzee titered stock of HIV-1 SF2, Boyer et al. further found, using a Reverse Transcriptase-Polymerase Chain Reaction (RT-PCR) assay, that those animals vaccinated with the DNA vaccine were protected against infection whereas the control animals were not so protected.

Kim et al., (1997 J. Immunol. 158:816-826), investigated the role of co-delivery of genes for IL-12 and GM-CSF along with DNA vaccine formulation for HIV-1 antigens env and gag/pol in mice. Kim et al. observed a dramatic increase in specific CTL response from the mice immunized with the HIV-1 DNA vaccine and IL-12. Kim et al. also observed that the co-delivery of IL-12 genes resulted in the reduction of specific antibody response, whereas the codelivery of GM-CSF genes resulted in the enhancement of specific antibody response. Kim et al. further observed that co-delivery of IL-12 gene with a HIV DNA vaccine results in splenomegaly (Kim et al. 1997, J. Immunol., 158:816-826), which has been shown in mice to have toxic effects such as weight reduction or even death (Eng et al., 1995, J. Exp. Med. 181:1893; Stevensen et al., 1995, J. Immunol. 155:2545; and Orange et al., 1995, J. Exp. Med. 181:901).

Notwithstanding the recent developments of the HIV DNA vaccine, there still exists a need for a method to enhance the efficacy of a vaccine, especially an HIV DNA vaccine. For instance, for efficacious vaccine against HIV-1 one preferably induces both cellular and humoral immune responses to control the infection (Boyer et al., 1997, Nature Medicine 3:625-532). The induction of both cellular and humoral immune response by the Boyer et al. method is still quite low because only one of the three immunized chimpanzees developed both cellular and humoral responses. Similarly, although co-delivery of an 1L-12 encoding gene with a HIV DNA vaccine, as described in Kim et al. (1997, J. Immun. 158:816-826), may have enhanced the cellular immune response, this co-delivery also decreased the humoral response.

Furci *et al*. describe an analysis of the HIV-specific T cell responses in exposed uninfected individuals who were either homozygous for the wild-type C-C chemokine receptor allele CCR-5 or heterozygous for the deletion allele. They found evidence of an oligoclonal T cell response mediated by helper T cells specific for a conserved region of the HIV-1 envelope.

WO96/40267 relates to a method of altering the specific, systemic immune response of an individual to a target antigen by the coadministration of a cytokine, an adhesion or accessory molecule, and the target antigen. The target antigen may be a tumour cell, a tumour cell antigen, an infectious agent or other foreign or non-foreign antigen, or other antigens to which an enhanced systemic immune response is desirable.

Citation of a reference hereinabove shall not be construed as an admission that such reference is prior art to the present invention.

### SUMMARY OF THE INVENTION.

The present invention is based upon the ability of chemokines, such as MDC, Rantes, BLC and MCP-1, to enhance the immune response to an antigen, particularly a vaccine. Accordingly, in a first aspect, the present invention provides a composition comprising: an immunogenic amount of a purified gap 120 or gp160 HIV glycoprotein antigen and an amount of a purified chemokine selected from the group consisting of MDC, BLC, RANTES and MCP-1, or purified fragments thereof, effective to enhance the immune response to said antigen; and a pharmaceutically acceptable carrier. The chemokine may be administered to a subject either concurrently with the purified antigen against which an immune response is desired or within a time period either before or after administration of the antigen such that the immune response against the antigen is enhanced.

In a second aspect, the present invention provides a composition comprising an amount of a first set of purified nucleic acids comprising a nucleotide sequence encoding a gp120 or gp160 HIV glycoprotein antigen and a second set of purified nucleic acids comprising a nucleotide sequence encoding a chemokine selected from the group consisting of MDC, BLC, RANTES and MCP-1, or fragments thereof, wherein the antigen and the chemokine, or fragment(s) thereof, are expressed from said first set of nucleic acids and second set of nucleic acids in a coordinated manner such that upon introduction into a suitable cell, the amount of said first set of nucleic acids is sufficient to express an immunogenic amount of the antigen and the amount of the said second set of nucleic acids is sufficient to express an amount of chemokine that is effective in enhancing the immune response to the HIV antigen; and a pharmaceutically acceptable carrier. This composition is effective to enhance the efficacy of a vaccine. The composition may be used in a method which comprises administration to a subject of a first set of purified nucleic acids comprising a nucleotide sequence encoding a chemokine selected from MDC, BLC, Rantes and MCP-1, or fragments or truncation isoforms thereof, and a second purified nucleic acid comprising a nucleotide sequence encoding a gp120 or gp160 HIV glycoprotein antigen against which an immune response is desired, such that, the chemokine and the antigen are expressed in a coordinated manner upon introduction into a suitable cell. Alternatively, the nucleotide sequences encoding the chemokine, or fragments thereof, and the antigen against which an immune response is desired are present on the same nucleic acid.

In a preferred embodiment, the invention provides a composition or use to enhance the immune response to HIV in a subject.

### 4. DESCRIPTION OF FIGURES

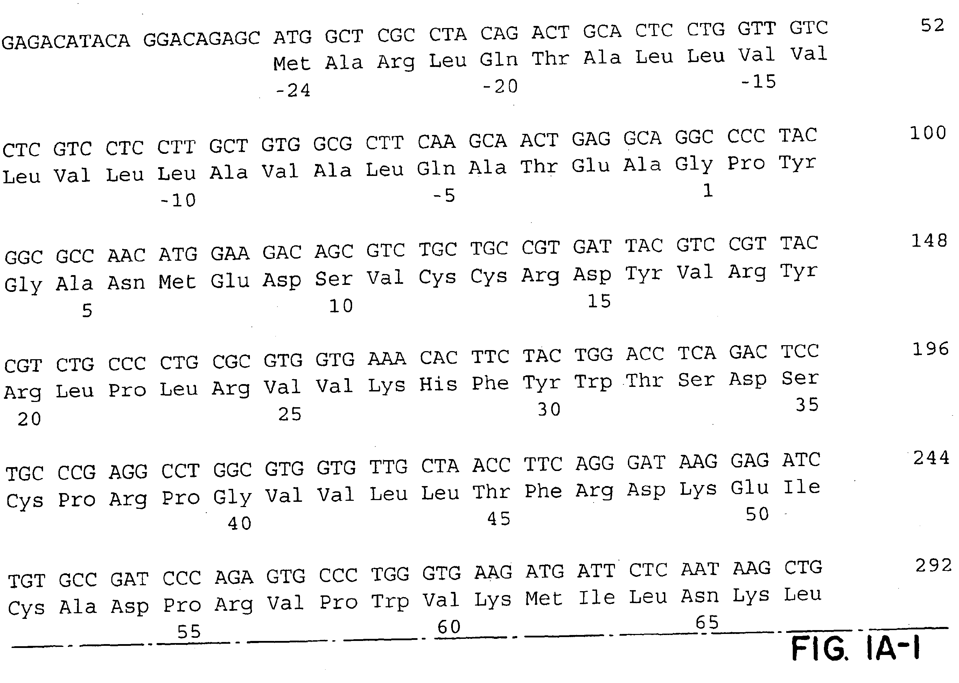

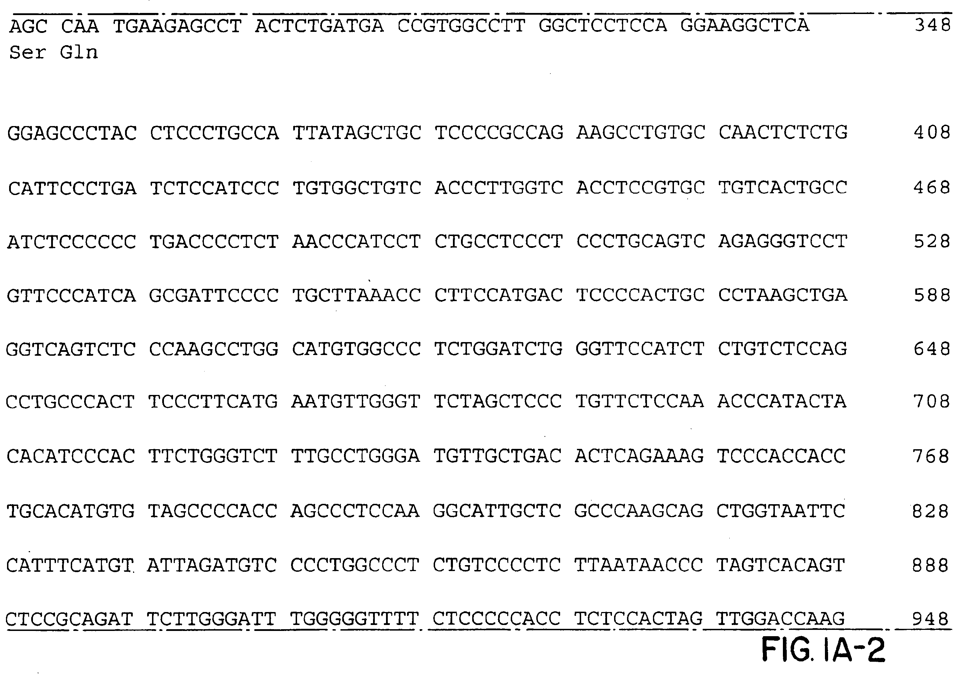

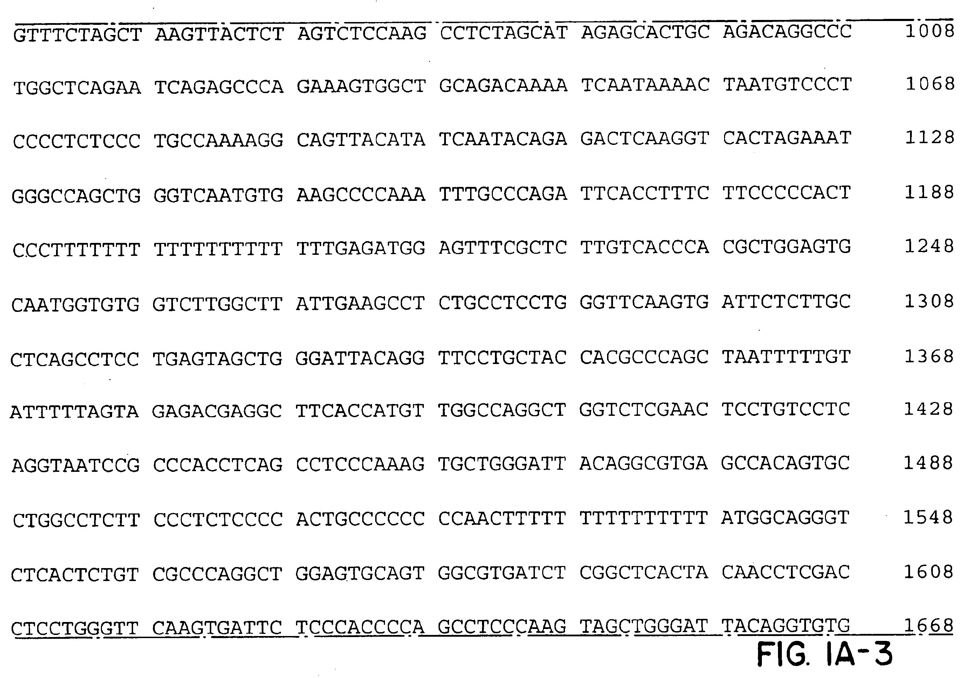

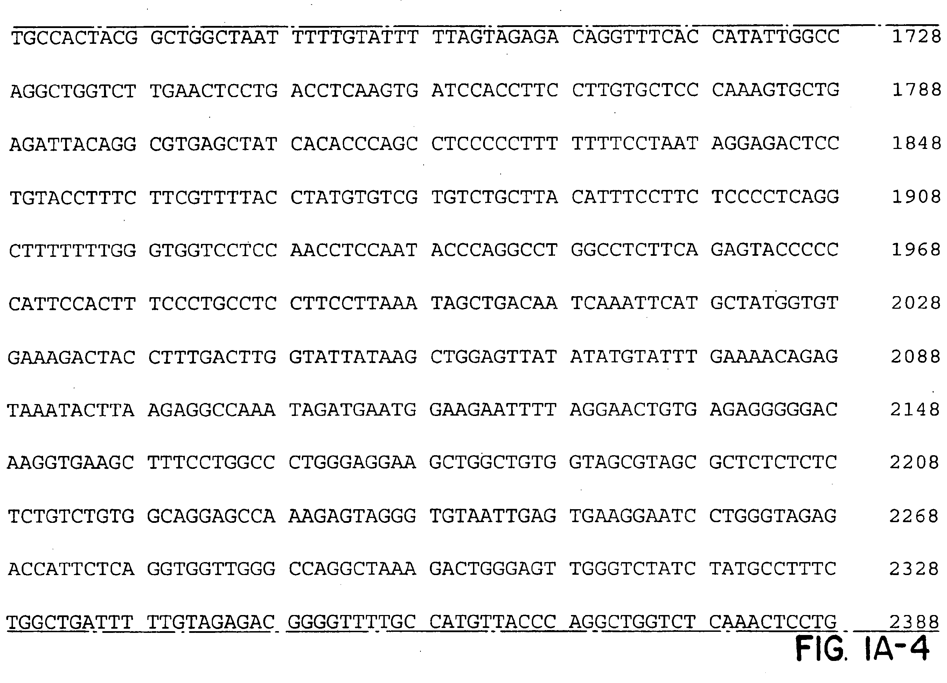

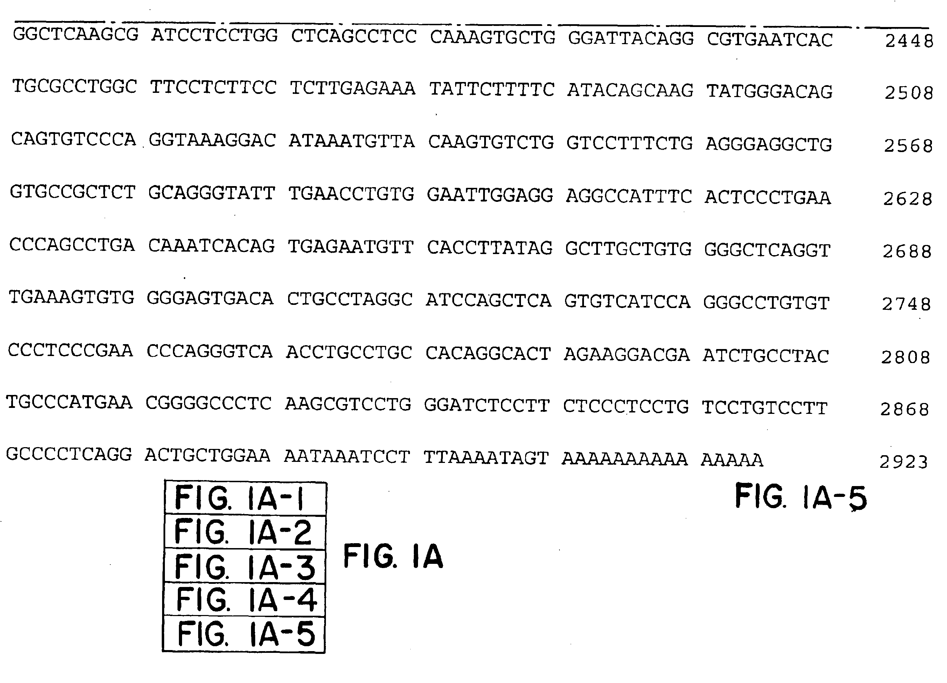

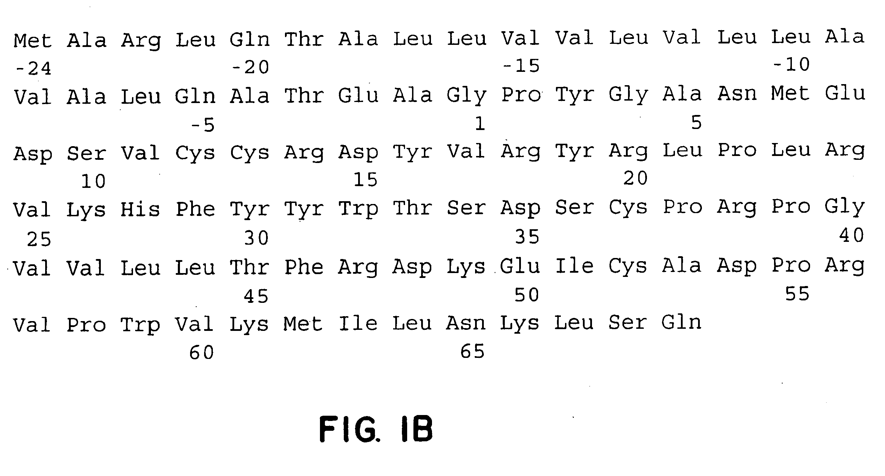

Figures 1A and 1B. The nucleotide and amino acid sequences of MDC. 1 A depicts the nucleotide sequence of MDC (SEQ ID NO:1), with the coding region indicated by the appearance of the amino acid sequence in the line below; and 1B depicts the amino acid of MDC (SEQ ID NO:2) from GenBank accession no. U83171 (Godiska et al., 1997, J. Exp. Med. 185:1595-1604).

### 5. DETAILED DESCRIPTION OF THE INVENTION

The present invention relates to compositions comprising a purified gp120 or gp160 HIV glycoprotein antigen and a purified chemokine, or fragment or truncation isoforms thereof.

The chemokine employed according to the present invention is selected from the following table:

| **Chemokine Class** | **Chemokines** | **Abbreviations** | **Accession Number** |
|---|---|---|---|
| **CC Chemokines** | Macrophage-derived chemokine | MDC/STCP-1 | u83171; u83239 |
| | Monocyte chemotactic protein 1 | MCP-1 | x14768 |
| | Regulated upon activation, normal T cell expressed and secreted (and its variants) | RANTES | M21211 |
| **CXC Chemokines** | B-cell-attracting chemokine 1 | BLC | AJ002211 |

The present invention also relates to the use of fragments of the foregoing chemokines, as well as truncation isoforms of such chemokines which are known in the art.

In another preferred embodiment of the invention, nucleic acids comprising nucleotide sequences encoding a chemokine selected from MDC, BLC, Rantes and MCP-1, or fragments, including truncation isoforms, thereof, and encoding an antigen against which an immune response is desired (HIV gp120 or gyp160), which coding sequences are operatively linked to gene regulatory sequences capable of directing the expression of the chemokine and the antigen upon introduction into a suitable cell, for example, but not limited to, the cell (of a subject), are administered to a subject such that the chemokine, or fragments, including truncation isoforms, thereof, and antigen, are expressed in the subject.

For clarity of disclosure, and not by way of limitation, the detailed description of the invention is divided into the subsections which follow.

### 5.1. METHODS AND COMPOSITIONS TO ENHANCE THE EFFICACY Of A VACCINE.

The compositions of the present invention may be used in methods for enhancing the efficacy of a vaccine in a subject, which methods comprise administering to a subject an immunogenic amount of a purified antigen against which an immune response is desired in the subject in conjunction with an amount of a purified chemokine, or fragments or truncation isoforms thereof, effective to enhance the immune response against the antigen. The purified chemokine, or fragment(s) thereof, may be administered to the subject concurrently with (e.g., in the same composition with) the purified antigen against which an immune response is desired. Alternatively, the purified chemokine, or fragment(s), or truncation isoforms thereof, may be administered either before or after the administration of the purified antigen against which immunity is desired in the subject, but is administered within such time that the chemokine enhances the immune response to the antigen. For example, but not by way of limitation, the purified chemokine may be administered during the time that the subject mounts an immune response against the administered antigen, or, the purified MDC is administered within, for example, but not limited to, 30 minutes, 1 hour, 5 hours, 10 hours, 1 day; 2 days of (preferably, after) administration of the purified antigens against which immunity is desired.

In a preferred embodiment, the present invention provides compositions comprising an immunogenic amount of a purified gp120 or gp160 HIV glycoprotein antigen and an amount of purified MDC, or one or more fragments, derivatives or analogues thereof, effective to enhance the immune response to said antigen and, preferably, the composition further comprises a pharmaceutically acceptable carrier.

A preferred chemokine for use in the methods and compositions of the present invention is any MDC protein, or fragment thereof, that is capable of enhancing the efficacy of a vaccine (for example, but not limited to, as determined by the assays described in Section 5.4, infro). In one specific embodiment, the MDC is purified full length. MDC, preferably full length MDC having the amino acid sequence of SEQ ID NO: 2 (Figure 1B). In another embodiment, the MDC is a purified protein, the amino acid sequence of which consists of amino acid numbers 2-69 of SEQ ID NO.: 2 (Figure 1B). In another specific embodiment, the MDC is a purified protein, the amino acid sequence of which consists of amino acid numbers 3-69 of SEQ ID NO: 2 (Figure 1 B). In still another specific embodiment, the MDC is a purified protein, the N-terminal amino acid sequence of which consists of the amino acid sequence Tyr-Gly-Ala-Asn-Met-Glu-Asp-Ser-Val-Cys-Cys-Arg-Asp-Tyr-Val-Arg-Tyr-Arg-Leu (portion of SEQ ID NO: 2), In yet another specific embodiment, the MDC is a purified protein, the N-terminal amino acid sequence of which consists of the amino acid sequence Pro-Tyr-Gly-Ala-Asn-Met-Glu-Asp-Ser-Val-Cys-Cys-Arg (portion of SEQ ID NO: 2). In yet another specific embodiment, the MDC is a purified derivative of a protein, the N-terminal amino acid sequence of which protein consists of the amino acid sequence Tyr-Gly-Ala-Asn-Met-Glu-Asp-Ser-Val-Cys-Cys-Arg-Asp-Tyr-Val-Arg-Tyr-Arg-Leu (SEQ ID NO:2), which derivative has activity to enhance the efficacy of the vaccine. In yet another specific embodiment, the MDC is a purified derivative of a protein, the N-terminal amino acid sequence of which protein consists of the amino acid sequence Pro-Tyr-Gly-Ala-Asn-Met-Glu-Asp-Ser-Val-Cys-Cys-Arg (SEQ ID NO:2), which derivative has activity to enhance the efficacy of the vaccine.

Preferably, the chemokine is of the same species as the subject to which the vaccine is administered. In a preferred embodiment, one or more human chemokines are administered to a human subject, e.g., human MDC is administered to a human subject.

The present invention also provides compositions to enhance the efficacy of a vaccine in a subject, which compositions comprise a purified first nucleic acid comprising a nucleotide sequence encoding a gp120 or gp160 HIV glycoprotein antigen and a purified second nucleic acid comprising a nucleotide sequence encoding a chemokine selected from MDC, BLC, RANTES and MCP-1, or fragments, including truncation isoforms thereof, wherein the nucleotide sequences encoding the antigen and the chemokine are operably linked to one or more gene regulatory elements such that, upon introduction of said first and second nucleic acids into a suitable cell (e.g., a cell of the subject), the antigen and chemokine are expressed in a coordinated manner and the antigen is expressed in an immunogenic amount and the chemokine is expressed in an amount effective to enhance the immune response against the antigen, relative to a corresponding immune response in the absence of such chemokine.

The present invention also provides compositions to enhance the efficacy of a vaccine in a subject, which compositions comprise a purified first set of one or more purified nucleic acids comprising a nucleotide sequence encoding a gp120 or gp160 HIV glycoprotein antigen and a purified second set of purified nucleic acids comprising a nucleotide sequence encoding a chemokine selected .from MDC, BLC, RANTES and MCP-1, or fragments (including truncation isoforms) thereof, wherein the nucleotide sequence(s) encoding the antigen and the chemokine are operably linked to one or more gene regulatory elements such that, upon introduction of said first and second sets of nucleic acids into a suitable cell (e.g., a cell of the subject), the antigen and chemokine are expressed in a coordinated manner and the antigen is expressed in an immunogenic mount and the chemokine is expressed in an amount effective to enhance the immune response against the antigen, relative to a corresponding immune response in the absence of such chemokine.

The present invention also provides compositions to enhance the efficacy of a vaccine in a subject, which compositions comprise a purified nucleic acid comprising a first set of one or more nucleotide sequences encoding a gp120 or gp160 HIV glycoprotein antigen and a second set of one ore more nucleotide sequence encoding a chemokine selected from MDC, BLC, RANTES and MCP-1, or fragments thereof (including truncation isoforms), wherein the first and second sets of nucleotide sequences are operably linked to one or more gene regulatory elements such that, upon introduction into a suitable cell, the antigen and the chemokine are expressed in a coordinated manner and the antigen is expressed in an immunogenic amount and the chemokine is expressed in an amount effective to enhance the immune response against the antigen (s).

Such compositions may be used in a method which comprises administering to a subject a purified first nucleic acid comprising a nucleotide sequence encoding a gp120 or gp160 HIV glycoprotein antigen against which an immune response is desired in a subject and a purified second nucleic acid comprising a nucleotide sequence encoding a chemokine selected from MDC, BLC, RANTES and MCP-1, or fragment(s) thereof, where the expression of the encoded antigen and chemokine, or fragment, are under control of one or more appropriate gene regulatory elements (which regulatory elements can be any regulatory element known in the art, for example, but not limited to, those regulatory elements described in Section 5.2 supra), such that, upon introduction of said first and second nucleic acids into a suitable cell (e.g., a cell of the subject), the antigen and chemokine, or fragment(s) thereof, are coordinately expressed, *i.e*., are expressed either at the same time or within an appropriate time period (*i.*e., sufficient for the chemokine(s) to enhance the immune response against the antigen relative to a corresponding immune response in the absence of the chemokine) and the antigen is expressed in an immunogenic amount and the chemokine, or fragment(s) thereof, are expressed in an amount sufficient to enhance the immune response against the antigen. The nucleotide sequences encoding the chemokine and the antigen may be present on separate nucleic acids. Alternatively, the nucleotide sequences encoding the chemokine and the antigen may be present on the same nucleic acid.

Any nucleic acid comprising a nucleotide sequence encoding a chemokine protein, or fragments thereof (including truncation isoforms), that is capable of enhancing the immune response to the antigen (for example, but not limited to, as determined by any of the assays described in Section 5.2., *infra*) can be used in the compositions of the present invention.

In a preferred embodiment, the nucleotide sequence encodes MDC. In another embodiment, the MDC-encoding nucleotide consists of the nucleotide sequence of SEQ ID NO: (Figure 1A).

Such compositions of nucleic acids encoding an antigen are often referred to as DNA vaccines.

Such DNA vaccines are produced by any method known in the art for constructing an expression plasmid vector containing the nucleotide sequences of the antigen(s) and/or cherhokine(s) to be expressed which vector is suitable for expression of the encoded proteins in the subject or in cells recombinant for the expression vector, which cells are to be provided to the subject. Such expression vectors may contain various promoters, terminators and polyadenylation coding regions to control the expression of the encoded protein.

The DNA vaccine can be administered by any method known in the art for administration of DNA. The DNA vaccine may be delivered either directly, in which case the subject is directly exposed to the DNA vaccine such that the DNA enters and is expressed in cells of the subject, or indirectly, in which case, the DNA vaccine is first introduced into suitable cells by any method known in the art *in vitro,* then the cells containing the DNA vaccine are transplanted into the subject.

The DNA vaccine may be directly administered *in vivo,* where it is expressed to produce the encoded antigens and chemokine(s). This can be accomplished by any of numerous methods known in the art, e.g., by constructing it as part of an appropriate nucleic acid expression vector and administering it so that it becomes intracellular, e.g., by infection using a defective or attenuated retroviral or other viral vector (see U.S. Patent No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules, or by administering it in linkage to a peptide which is known to enter the nucleus, by administering it in linkage to a ligand subject to receptor-mediated endocytosis (see e.g., Wu and Wu, J. Biol. Chem. 262:4429-4432 (1987)) (which can be used to target cell types specifically expressing the receptors), etc. Alternatively, a nucleic acid-ligand complex can be formed in which the ligand comprises a fusogenic viral peptide to disrupt endosomes, allowing the nucleic acid to avoid lysosomal degradation. Preferably, the nucleic acid of a DNA vaccine may be injected into the muscle of the subject to be immunized.

Another approach is to introduce the nucleic acid of the DNA vaccine into a cell prior to administration in vivo of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microceH-mediated gene transfer, spheroplast fusion, etc. Numerous techniques are known in the art for the introduction of foreign nucleic acid into cells (see e.g., Loeffler and Behr, Meth. Enzymol. 217:599-618 (1993); Cohen et al., Meth. Enzymol. 217:618-644 (1993); Cline, Pharmac. Ther. 29:69-92 (1985)). Usually, the method of transfer includes the transfer of a selectable marker to the cells. The cells are then placed under selection to isolate those cells that have taken up and are expressing the transferred gene.

Cells into which a DNA vaccine can be introduced for purposes of immunization encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc.

The resulting recombinant cells can be delivered to a subject by various methods known in the art. The recombinant cells may be injected, e.g., subcutaneously. Alternatively, recombinant skin cells may be applied as a skin graft onto the patient. Recombinant blood cells (e.g., hematopoietic stem or progenitor cells) are preferably administered intravenously. The cells can also be encapsulated in a suitable vehicle and then implanted in the subject (see, *e.g*., Dionne et al. PCT Publication WHO 92/19195, dated November 12, 1992). The amount of cells envisioned for use depends on the desired effect, subject state, etc., and can be determined by one skilled in the art.

By way of example, and not by way of limitation a DNA vaccine may be generated as described by Lekutis et al. for an HIV DNA vaccine (1997, J. immunol, 158:4471-4477). Briefly, an expression vector, is constructed with the promoter, enhancer and intron A of human cytomegalovirus (CMV) and the termination and polyadenylation sequences of bovine growth hormone in a plasmid backbone. Additionally, the nucleotide sequence for signal sequence of tissue plasminogen activator is either substituted for the signal sequence of the antigen, if the antigen has a signal sequence or is added onto the amino-terminus of the antigen, thereby eliminating the dependence on viral proteins for expression (e.g., in the case of gp120 expression, rev and env proteins are required unless the HIV-1 signal sequence is so substituted). The resulting formulation is then injected intramuscularly.

Further examples of DNA vaccines are set forth in Boyer et al. (1996, J. Med. Primatol., 25:242-250), which describes the construction of a plasmid encoding the HIV-1 gp160 envelope glycoprotein as well as the *rev-tax* region cloned into pMAMneoBlue vector (Clonetech, Inc., Palo Alto, CA), and a vector encoding the envelope glycoprotein and rev from HIV-1 strain MN under the control of the CMV promoter. Another vector which can be used in the present invention is as described in Boyer et al. (1997, Nature Medicine 3:526-532) and contains expression cassettes encoding the envelope and Rev proteins of HIV-1 strain MN, and encoding the Gag/Pol proteins of HIV-1 strain IIIB.

For the practice of the present invention, the nucleotide sequence for the one or more chemokines, or fragments, derivatives, or analogues thereof, can either be incorporated into the same expression vector containing the nucleotide sequence encoding the antigen in such a manner that the chemokine(s) are expressed. Alternatively, the nucleotide sequence encoding the chemokine(s), or fragment(s) thereof, can be cloned into a separate expression vector (e.g.. as described above for the expression vector containing the sequences coding for antigen) and the expression vector that expresses the antigen(s) mixed with the expression vector that expresses the chemokine(s). The mixture of the two expression vectors can then be administered to the subject.

The compositions of the present invention may be used as a vaccine in a subject in which immunity for the antigen is desired. The antigen is gp120 or gp160 HIV glycoprotein antigen. The composition of the present invention may be used to enhance an immune response to HIV. In a preferred embodiment, the antigen is an HIV-associated gp120 protein.

The compositions of the present invention may be used to elicit as humoral and/or a cell-mediated response against the antigen of the vaccine in a subject. In one specific embodiment, the compositions elicit a humoral response against the administered antigen in a subject. In another specific embodiment, the compositions elicit a cell-mediated response against the administered antigen in a subject. In a preferred embodiment, the compositions elicit both a humoral and a cell-mediated response.

The subjects to which the present invention is applicable may be any mammalian or vertebrate species, which include, but are not limited to, cows, horses, sheep, pigs, fowl (e.g., chickens), goats, cats, dogs, hamsters, mice and rats; monkeys, rabbits, chimpanzees, and humans. In a preferred embodiment, the subject is a human. The compositions of the invention can be used to either prevent or to treat HIV, where an immune response against a particular antigen is effective to treat or prevent the HIV. In a preferred embodiment, the subject is infected or at risk of being infected with HIV virus.

In another preferred embodiment the invention provides compositions to enhance the efficacy of an HIV vaccine, such a vaccine can be administered to either prevent or treat HIV.

### 5.2. CHEMOKINE GENES AND PROTEINS

Chemokine proteins and nucleic acids can be obtained by any method known in the art. Chemokine nucleotide and amino acid sequences are available in public databases such as Genbank and are also published in various references known to those of skill in the art. The gene bank accession numbers for the preferred chemokines of the present invention are provided in Table 1, in Section 5 above. The ensuing discussion uses MDC by way of example, but applies equally to other chemokines as well.

The MDC nucleotide and amino acid sequences for, inter alia, human, are available in the public databases (*e.g.* Genbank accession No. U83171) also published in Godiska et al., 1997, J. Exp. Med. 185:1595-1604. The nucleotide sequence and the amino acid sequence for the human MDC are provided in Figures 1A and B (SEQ ID NOS:1 and 2, respectively).

Chemokines used herein include, but are not limited to, chemokines from mice, hamsters, dogs, cats, monkeys, rabbits, chimpanzees, and human. In one preferred embodiment, the chemokine is of human origin.

Any vertebrate cell potentially can serve as the nucleic acid source for the isolation of chemokine nucleic acids. The nucleic acid sequences encoding the chemokine(s) can be isolated from vertebrate, mammalian, human, porcine, bovine, feline, avian, equine, canine, as well as additional primate sources, etc. The DNA may be obtained by standard procedures known in the art from cloned DNA (*e.g.*, a DNA "library"), by chemical synthesis, by cDNA cloning, or by the cloning of genomic DNA, or fragments thereof, purified from the desired cell (see, for example, Sambrook et al., 1989, Molecular Cloning, A Laboratory Manual, 2d Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II.) Clones derived from genomic DNA may contain regulatory and intron DNA regions in addition to coding regions; clones derived from cDNA will contain only exon sequences. Whatever the source, the gene should be molecularly cloned into a suitable vector for propagation of the gene.

In the molecular cloning of the gene from cDNA, cDNA is generated from totally cellular RNA or mRNA by methods that are well known in the art. The gene may also be obtained from genomic DNA, where DNA fragments are generated (e.g. using restriction enzymes or by mechanical shearing), some of which will encode the desired gene. The linear DNA fragments can then be separated according to size by standard techniques, including but not limited to, agarose and polyacrylamide gel electrophoresis and column chromatography.

Once the DNA fragments are generated, identification of the specific DNA fragment containing all or a portion of the chemokine gene may be accomplished in a number of ways.

A preferred method for isolating a chemokine gene is by the polymerase chain reaction (PCR), which can be used to amplify the desired chemokine sequence in a genomic or cDNA library or from genomic DNA or cDNA that has not been incorporated into a library. Oligonucleotide primers which would hybridize to chemokine sequences can be used as primers in PCR.

Additionally, a portion of the chemokine (of any species) gene or its specific RNA, or a fragment thereof, can be purified (or an oligonucleotide synthesized) and labeled, the generated DNA fragments may be screened by nucleic acid hybridization to the labeled probe (Benton, W. and Davis, R., 1977, Science 196:180; Grunstein, M. And Hogness, D., 1975, Proc. Natl. Acad. Sci. U.S.A. 72:3961). Those DNA fragments with substantial homology to the probe will hybridize. Chemokine nucleic acids can be also identified and isolated by expression cloning using, for example, anti-chemokine antibodies for selection.

Alternatives to obtaining the chemokine DNA by cloning or amplification include, but are not limited to, chemically synthesizing the gene sequence itself from the known chemokine sequence or making cDNA to the mRNA which encodes the chemokine protein. Other methods are possible and within the scope of the invention. Once a clone has been obtained, its identity can be confirmed by nucleic acid sequencing (by any method well known in the art) and comparison to known chemokine sequences. DNA sequence analysis can be performed by any techniques known in the art, including but not limited to the method of Maxam and Gilbert (1980, Meth. Enzymol. 65:499-560), the Sanger dideoxy method (Sanger, F., et al., 1977, Proc. Natl. Acad. Sci. U.S.A. 74:5463), the use of T7 DNA polymerase (Tabor and Richardson, U.S. Patent No. 4,795,699), use of an automated DNA sequenator (e.g., Applied Biosystems, Foster City, CA) or the method described in PCT Publication WO 97/ 15690.

Nucleic acids which are hybridizable to a chemokine nucleic acid, or to a nucleic acid encoding a chemokine derivative can be isolated, by nucleic acid hybridization under conditions of low, high, or moderate stringency (see also Shilo and Weinberg, 1981, Proc. Natl. Acad. Sci. USA 78:6789-6792). For example, the nucleic acid of SEQ ID No: 1 is hybridizable to an MDC nucleic acid.

Chemokine proteins and derivatives, analogs and fragments of chemokine proteins can be obtained by any method known in the art, including but not limited to recombinant expression methods, purification from natural sources, and chemical synthesis.

For example, chemokines can be obtained by recombinant protein expression techniques. For recombinant expression, the chemokine gene or portion thereof is inserted into an appropriate cloning vector for expression in a particular host cell. A large number of vector-host systems known in the art may be used. Possible vectors include, but are not limited to, plasmids or modified viruses, but the vector system must be compatible with the host cell used. Such vectors include, but are not limited to, bacteriophages such as lambda derivatives, or plasmids such as pBR322 or pUC plasmid derivatives or the Bluescript vector (Stratagene). The insertion into a cloning vector can, for example, be accomplished by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. In an alternative method, the cleaved vector and chemokine gene may be modified by homopolymeric tailing. Recombinant molecules can be introduced into host cells via transformation, transfection, infection, electroporation, etc., so that many copies of the gene sequence are generated.

In an alternative method, the desired gene may be identified and isolated after insertion into a suitable cloning vector in a "shot gun" approach. Enrichment for the desired gene, for example, by size fractionation, can be done before insertion into the cloning vector.

In specific embodiments, transformation of host cells with recombinant DNA molecules that incorporate the isolated chemokine gene, cDNA, or synthesized DNA sequence enables generation of multiple copies of the gene. Thus; the gene may be obtained in large quantities by growing transformants, isolating the recombinant DNA molecules from the transformants and, when necessary, retrieving the inserted gene from the isolated recombinant DNA.

The nucleotide sequence coding for a chemokine protein or a functionally active analog or fragment or other derivative thereof, can be inserted into an appropriate expression vector, *i.e.*, a vector which contains the necessary elements for the transcription and translation of the inserted protein-coding sequence. The necessary transcriptional and translational signals can also be supplied by the native chemokine gene and/or its flanking regions. A variety of host-vector systems may be utilized to express the protein-coding sequence. These include but are not limited to mammalian cell systems infected with virus (*e.g.*, vaccinia virus, adenovirus, etc.); insect cell systems infected with virus (*e.g.*, baculovirus); microorganisms such as yeast containing yeast vectors, or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

Any of the methods previously described for the insertion of DNA fragments into a vector may be used to construct expression vectors containing a chimeric gene consisting of appropriate transcriptional/translational control signals and the protein coding sequences. These methods may include *in vitro* recombinant DNA and synthetic techniques and *in vivo* recombinants (genetic recombination). Expression of nucleic acid sequence encoding a chemokine protein or peptide fragment may be regulated by a second nucleic acid sequence so that the chemokine protein or peptide is expressed in a host transformed with the recombinant DNA molecule. For example, expression of a chemokine protein may be controlled by any promoter/enhancer element known in the art. Promoters which may be used to control chemokine expression include, but are not limited to, the SV40 early promoter region (Bernoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); prokaryotic expression vectors such as the β-lactamase promoter (Villa-Kamaroff, et al., 1978, Proc. Natl. Acad. Sci. U.S.A. 75:3727-3731), or the *tac* promoter (DeBoer, et al., 1983, Proc. Natl. Acad. Sci. U.S.A. 80:21-25); see also "Useful proteins from recombinant bacteria" in Scientific American, 1980, 242:74-94; promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell . 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94), myelin basic protein gene control region which is active in oligodendrocyte cells in the brain (Readhead et al., 1987, Cell 48:703-712), myosin light chain-2 gene control region which is active in skeletal muscle (Sani, 1985, Nature 314:283-286), and gonadotropic releasing hormone gene control region which is active in the hypothalamus (Mason et al., 1986, Science 234:1372-1378).

For example, a vector can be used that comprises a promoter operably linked to an chemokine-encoding nucleic acid, one or more origins of replication, and, optionally, one or more selectable markers (*e.g.*, an antibiotic resistance gene).

In a specific embodiment, an expression construct is made by subcloning a chemokine coding sequence into the EcoRI restriction site of each of the three pGEX vectors (Glutathione S-Transferase expression vectors; Smith and Johnson, 1988, Gene 7:31-40). This allows for the expression of the chemokine protein product from the subclone in the correct reading frame.

Expression vectors containing chemokine gene inserts can be identified by three general approaches: (a) nucleic acid hybridization, (b) presence or absence of "marker" gene functions, and (c) expression of inserted sequences. In the first approach, the presence of a chemokine gene inserted in an expression vector can be detected by nucleic acid hybridization using probes comprising sequences that are homologous to an inserted chemokine gene. In the second approach, the recombinant vector/host system can be identified and selected based upon the presence or absence of certain "marker" gene functions (*e.g.*, thymidine kinase activity, resistance to antibiotics, transformation phenotype, occlusion body formation in baculovirus, etc.) caused by the insertion of a chemokine gene in the vector. For example, if the chemokine gene is inserted within the marker gene sequence of the vector, recombinants containing the chemokine insert can be identified by the absence of the marker gene function. In the third approach, recombinant expression vectors can be identified by assaying the product expressed by the recombinant. Such assays can be based, for example, on the physical or functional properties of the chemokine protein in *in vitro* assay systems, *e.g.*, binding with anti-chemokine antibody or the chemokine's receptor.

Once a particular recombinant DNA molecule is identified and isolated, several methods known in the art may be used to propagate it. Once a suitable host system and growth conditions are established, recombinant expression vectors can be propagated and prepared in quantity. As previously explained, the expression vectors which can be used include, but are not limited to, the following vectors or their derivatives: human or animal viruses such as vaccinia virus or adenovirus; insect viruses such as baculovirus; yeast vectors; bacteriophage vectors (*e.g.*, lambda), and plasmid and cosmid DNA vectors, to name but a few.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered protein may be controlled. Furthermore, different host cells have characteristic and specific mechanisms for the translational and post-translational processing and modification (*e.g.*, glycosylation, phosphorylation of proteins. Appropriate cell lines or host systems can be chosen to ensure the desired modification and processing of the foreign protein expressed. For example, expression in a bacterial system can be used to produce an unglycosylated core protein product. Expression in yeast will produce a glycosylated product. Expression in mammalian cells can be used to ensure "native" glycosylation of a heterologous protein. Furthermore, different vector/host expression systems may effect processing reactions to different extents.

In other specific embodiments, the chemokine protein(s), fragment(s), analogue(s), or derivative(s) may be expressed as a fusion, or chimeric protein product (comprising the protein, fragment, analog, or derivative joined via a peptide bond to a heterologous protein sequence (of a different protein)). Such a chimeric product can be made by ligating the appropriate nucleic acid sequences encoding the desired amino acid sequences to each other by methods known in the art, in the proper coding frame, and expressing the chimeric product by methods commonly known in the art. Alternatively, such a chimeric product may be made by protein synthetic techniques, *e.g.*, by use of a peptide synthesizer. In a specific embodiment, a chimeric protein containing all or a portion of the chemokine is joined via a peptide bond to all or a portion of an antigen against which immunity is desired.

Both cDNA and genomic sequences can be cloned and expressed.

The chemokine protein(s) may also be isolated and purified by standard methods including chromatography (*e.g.*, ion exchange, affinity, and sizing column chromatography), centrifugation, differential solubility, or by any other standard technique for the purification of proteins. The functional properties may be evaluated using any suitable assay (see Section 5.5). Alternatively, the protein can be synthesized by standard chemical methods known in the art (*e.g.*, see Hunkapiller, M., et al., 1984, Nature 310:105-111). The chemokine-encoding nucleic acid sequence(s) can be mutated *in vitro* or *in vivo,* to create and/or destroy translation, initiation, and/or termination sequences, or to create variations in coding regions. Any technique for mutagenesis known in the art can be used, including, but not limited to, *in vitro* site-directed mutagenesis (Hutchinson et al., 1978, J. Biol. Chem 253:6551), use of TAB linkers (Pharmacia), mutation-containing PCR primers, etc.

The experimentation involved in mutagenesis consists primarily of site-directed mutagenesis followed by phenotypic testing of the altered gene product. Some of the more commonly employed site-directed mutagenesis protocols take advantage of vectors that can provide single stranded as well as double stranded DNA, as needed. Generally, the mutagenesis protocol with such vectors is as follows. A mutagenic primer, *i.e.*, a primer complementary to the sequence to be changed, but consisting of one or a small number of altered, added, or deleted bases, is synthesized. The primer is extended *in vitro* by a DNA polymerase and, after some additional manipulations, the now double-stranded DNA is transfected into bacterial cells. Next, by a variety of methods, the desired mutated DNA is identified, and the desired protein is purified from clones containing the mutated sequence. For longer sequences, additional cloning steps are often required because long inserts (longer than 2 kilobases) are unstable in those vectors. Protocols are known to these skilled in the art and kits for site-directed mutagenesis are widely available from biotechnology supply companies, for example from Amersham Life Science, Inc. (Arlington Heights, IL) and Stratagene Cloning Systems (La Jolla, CA).

In other specific embodiments, the chemokine derivative(s) or analogue(s) may be expressed as a fusion, or chimeric protein product (comprising the protein, fragment, analogue, or derivative joined via a peptide bond to a heterologous protein sequence (of a different protein)). Such a chimeric product can be made by ligating the appropriate nucleic acid sequences encoding the desired amino acid sequences to each other by methods known in the art, in the proper coding frame, and expressing the chimeric product by methods commonly known in the art.

In addition, chemokine proteins, derivatives (including fragments and chimeric proteins), and analogues can be chemically synthesized. See, *e.g.*, Clark-Lewis et al., 1991, Biochem. 30:3128-3135 and Merrifield, 1963, J. Amer. Chem. Soc. 85:2149-2156. For example, chemokines, derivatives and analogues can be synthesized by solid phase techniques, cleaved from the resin, and purified by preparative high performance liquid chromatography (*e.g.*, see Creighton, 1983, Proteins, Structures and Molecular Principles, W.H. Freeman and Co., N.Y., pp. 50-60). Chemokines, derivatives and analogues that are proteins can also be synthesized by use of a peptide synthesizer. The composition of the synthetic peptides may be confirmed by amino acid analysis or sequencing (*e.g.*, the Edman degradation procedure; see Creighton, 1983, Proteins, Structures and Molecular Principles, W.H. Freeman and Co., N.Y., pp. 34-49).

The chemokine proteins, derivatives, or analogues of the invention may be synthesized in their entirety by the sequential addition of amino acid residues or alternatively as fragment subcomponents which may be combined using techniques well known in the art, such as, for example, fragment condensation (Shin et al., 1992, Biosci. Biotech. Biochem. 56:404-408; Nyfeler et al., 1992, Peptides, Proc. 12th Amer. Pep. Soc., Smith and Rivier (eds). Leiden, pp 661-663): and Nokihara et al., 1990, Protein Research Foundation, Yanaihara (ed), Osaka, pp 315-320).

In a less preferred embodiment, chemokine derivatives can be obtained by proteolysis of the protein followed by purification using standard methods such as those described above (*e.g.*, immunoaffinity purification).

In another alternate embodiment, native chemokine proteins can be purified from natural sources, by standard methods such as those described above (*e.g.*, immunoaffinity purification).

### 5.3. COMPOSITION FORMULATIONS AND METHODS OF ADMINISTRATION

The composition formulations of the invention comprise an effective immunizing amount of an immunologically active ingredient, i.e., an antigen, and an amount of a chemokine, or fragment(s) thereof, effective to enhance the immune response against the antigen in a subject, and a pharmaceutically acceptable carrier or excipient. The chemokines are selected from the group consisting of Macrophage-derived chemokine, Monocyte chemotactic protein 1; Regulated upon activation, normal T cell expressed and secreted (and its variants), and B-cell-attracting chemokine 1.

Pharmaceutically acceptable carriers or excipients are well known in the art and include but are not limited to saline, buffered saline, dextrose, water, glycerol, ethanol, sterile isotonic aqueous buffer, and combinations thereof. One example of such an acceptable carrier is a physiologically balanced culture medium containing one or more stabilizing agents such as stabilized, hydrolyzed proteins, lactose, etc. The carrier is preferably sterile. The formulation should suit the mode of administration.

In addition, if desired, the vaccine or composition preparation may also include- minor amounts of auxiliary substances such as wetting or emulsifying agents, pH buffering agents, and/or adjuvants which enhance the effectiveness of the vaccine or composition. Suitable adjuvants may include, but are not limited to: mineral gels, *e.g.*, aluminum hydroxide; surface active substances such as lysolecithin, pluronic polyols; polyanions; peptides; oil emulsions; alum, MDC, N-acetyl-muramyl-L-threonyl-D-isoglutamine (thr-MDP), N-acetyl-nor-muramyl-L-alanyl-D-isoglutamine, and N-acetylmuramyl-L-alanyl-D-isoglutaminyl-L-alanine-2-(1'-2'-dipalmitoyl-sn-glycero-3-hydroxyphosphoryloxy)-ethylamine. The effectiveness of an adjuvant may be determined by comparing the induction of antibodies directed against a MDC-containing composition in the presence and in the absence of various adjuvants.

In instances where the recombinant antigen is a hapten, *i.e.*, a molecule that is antigenic in that it can react selectively with cognate antibodies, but not immunogenic in that it cannot elicit an immune response, the hapten may be covalently bound to a carrier or immunogenic molecule; for instance, a large protein such as serum albumin will confer immunogenicity to the hapten coupled to it. The hapten-carrier may be formulated for use as a vaccine.

The composition can be a liquid solution, suspension, emulsion, tablet, pill, capsule, sustained release formulation, or powder. Oral formulation can include standard carriers such as pharmaceutical grades of mannitol, lactose, starch, magnesium stearate, sodium saccharine, cellulose, magnesium carbonate, etc.

The chemokine, or fragment(s) thereof, and/or the antigen may be formulated into the composition as neutral or salt forms. Pharmaceutically acceptable salts include the acid addition salts (formed with free amino groups of the peptide) and which are formed with inorganic acids, such as, for example, hydrochloric or phosphoric acids, or organic acids such as acetic, oxalic, tartaric, maleic, and the like. Salts formed with free carboxyl groups may also be derived from inorganic bases, such as, for example, sodium potassium, ammonium, calcium, or ferric hydroxides, and such organic bases as isopropylamine, trimethylamine, 2-ethylamino ethanol, histidine, procaine and the like.

The vaccines of the invention may be multivalent or univalent. Multivalent vaccines are made from recombinant viruses that direct the expression of more than one antigen.

An effective dose (immunizing amount) is that amount sufficient to produce an immune response to the antigen(s) in the host to which the vaccine preparation is administered. The precise dose of the composition to be employed in the formulation will depend on the route of administration, and the nature of the subject to be immunized, and should be decided by the practitioner according to standard clinical techniques. Effective doses of the vaccines or compositions of the present invention may also be extrapolated from dose-response curves derived from animal model test systems.

Containers comprising one or more of the ingredients of the composition formulations of the invention may be associated with a notice in the form prescribed by a governmental agency regulating the manufacture, use or sale of pharmaceuticals or biological products, which notice reflects approval by the agency of manufacture, use or sale for human administration.

Generally, the ingredients are supplied either separately or mixed together in unit dosage form, for example, as a dry lyophilized powder or water free concentrate in a hermetically sealed container such as an ampoule or sachette indicating the quantity of active agent. Where the composition is administered by injection, an ampoule of sterile diluent can be provided so that the ingredients may be mixed prior to administration.

A lyophilized immunologically active ingredient and one or more chemokine polypeptide(s) of the invention may be provided in a first container; a second container comprises diluent consisting of an aqueous solution of 50% glycerin, 0,25% phenol, and an antiseptic (*e.g.*, 0.005% brilliant green).

Many methods may be used to introduce the composition formulations of the invention; these include but are not limited to oral, intradermal, intramuscular, intraperitoneal, intravenous, subcutaneous, intranasal routes, and via scarification (scratching through the top layers of skin, *e.g.*, using a bifurcated needle).

The DNA vaccines of the invention can be administered by any method known in the art for delivery of DNA to subject (for example, as described in Section 5.3 supra)

### 5.4. DETERMINATION OF COMPOSITION EFFICACY

The activity of one or more chemokines, or a fragment, derivative or analogue thereof, to enhance immune response to an antigen can be determined by monitoring the immune response in test animals following immunization with a composition containing the chemokine(s) and an antigen and comparing the response to that following immunization with the antigen in the absence of the chemokine(s). Generation of a humoral (antibody) response and/or cell-mediated immunity, may be taken as an indication of an immune response. Test animals may include mice, hamsters, dogs, cats, monkeys, rabbits, chimpanzees, etc., and eventually human subjects. Assays for humoral and cell-mediated immunity are well known in the art.

Methods of introducing the composition may include oral, intracerebral, intradermal, intramuscular, intraperitoneal, intravenous, subcutaneous, intranasal or any other standard routes of immunization. The immune response of the test subjects can be analyzed by various approaches well known in the art, such as but not limited to: testing the reactivity of the resultant immune serum to the antigen of the chemokine-containing vaccine, as assayed by known techniques, *e.g.*, immunosorbant assay (ELISA), immunoblots, radioimmunoprecipitations, etc.

As one example of suitable animal testing, a composition of the present invention may be tested in mice for the ability to enhance an antibody response to an antigen (using for example, but not limited to, the method as described in Section 6, *infra*) and the delayed-type hypersensitivity (DTH) response (also described in Section 6 infra), measured by an increase in footpad swelling after inoculation in the footpad of the test animal, as compared to the measurements in animals administered the antigen in a composition not containing chemokine. For example, as test animals BALB/c mice may be used. The test group each receives an inoculation with fixed amount of antigen and varying amount of one or more chemokines. The control group receives an inoculation of comparable amount of antigen alone.

Serum samples may be drawn from the mice after the final inoculation (for example every one or two weeks after inoculation), and serum is analyzed for antibodies against the antigen using known methods in the art, *e.g.*, using an ELISA. DTH responses to the antigen may be measured after the final inoculation (e.g. within 1-7 days). An increase in the serum titer of antibodies recognizing the antigen and/or an increase in footpad swelling in the animals receiving the antigen-compositions containing the chemokine(s) as compared to the serum titer of antibodies against the antigen and/or the footpad swelling in the animals receiving the antigen composition not containing the chemokine(s), indicates that the chemokine(s) enhance the immune response to antigen. An increase in the serum titer of antibodies recognizing the antigen and/or an increase in footpad swelling in the animals receiving the antigen-compositions containing the chemokines as compared to the serum titer of antibodies against the antigen and/or the footpad swelling in the animals receiving the antigen composition not containing chemokine(s), indicates that the chemokine(s) enhances the immune response to antigen. An increase in the serum titer of antibodies recognizing the antigen and/or an increase in footpad swelling in the animals receiving the antigen-compositions containing MDC as compared to the serum titer of antibodies against the antigen and/or the footpad swelling in the animals receiving the antigen composition not containing MDC, indicates that the MDC enhances the immune response to antigen. An increased in the serum titer of antibodies recognizing the antigen and/or an increase in footpad swelling in the animals receiving the antigen-compositions containing MDC as compared to the serum titer of antibodies against the antigen and/or the footpad swelling in the animals receiving the antigen composition not containing MDC, indicates that the MDC enhances the immune response to antigen.

### 6. EXAMPLE: IMMUNIZATION WITH MDC-CONTAINING COMPOSITION

The following experiment illustrates the evaluation of whether MDC will act as an adjuvant for a protein antigen and enhance the efficacy of a vaccine. However, it will be appreciated that the description applies equally to other chemokines.

### 6.1. MATERIALS AND METHODS

### 6.1.1. ANIMALS AND REAGENTS

BALB/c mice are purchased from Harlan-Sprague-Dawley (Indianapolis, IN). Human MDC (hMDC) was obtained from CD8⁺, T cell clones immortalized *in vitro* prepared as previously described (Markham et al., 1983 Int. J. Cancer 31:413; Markham et al. 1984, Int. J. Cancer 33:13). One such immortalized CD8⁺ T cell clone, F3b Clone 19, was adapted to growth in serum-free medium by the following procedure and used for further studies. F3b Clone 19 cells were grown in complete medium containing rlL-2 (16 ng/ml) at 37°C in a CO₂ incubator. After expanding the culture to 200 ml, the cells were pelleted and resuspended in RPMI medium containing HB101 (Irvine Scientific) supplemented with 16 ng/ml of rIL-2, 1% glutamine and 1% penicillin/streptomycin. The cells were grown to full confluence and the medium harvested by centrifugation at 670 x g for 10 minutes.

Human MDC (hMDC) was purified from F3b Clone 19 as described in Pal et al., 1997, Science 278:695-698. Briefly, the cell free culture supernatant from F3b Clone 19 was clarified by high speed centrifugation and fractionated by heparin affinity chromatography, taking advantage of the heparin binding characteristics of chemokines (Witt and Lander, 1994, Current Biology 4:394; Proost et al., 1996, Method: A Companion to Methods in Enzymology 10:82). Culture supernatant (1200 ml) from F3b Clone 19, grown to high cell density in serum-free medium supplemented with rIL-2 was clarified by high speed centrifugation (100,000 x g for 60 minutes at 4°C) and applied to a 5 ml HiTrap heparin affinity FPLC column (Pharmacia) equilibrated in 10 mM Tris-HCl, pH 7.6 containing 0.1 M NaCl (column buffer). The column was then washed extensively with column buffer and the bound proteins eluted from the column with 10 mM Tris-HCl, pH 7.6 containing 2.0 M NaCl at a flow rate of 0.5 to 1 ml/minute. Virtually all of the HIV suppressive activity effective against primary NSI and SI isolates and HIV-1 _{IIIB} was recovered in the column eluate (data not shown). The heparin affinity column eluate was brought to pH 2.0 by addition of trifluoracetic acid (TFA) and subjected to reversed phase HPLC on a PEEK C-18 column (Waters Instruments) equilibrated in H₂O containing 0.1 % TFA. Proteins bound to the column were eluted with a 5 minute linear gradient of aqueous acetonitrile (0 to 35 %) containing 0.1% TFA. After 10 minutes at 35% acetonitrile, the column was further developed with a 60 minute linear gradient of 35-70% aqueous acetonitrile in TFA. The flow rate was maintained at 0.5 to 1 ml/minute. The fractions obtained were then tested for suppressor activity in the acute infectivity assay using HIV-1_{IIIB}. Active fractions were pooled, diluted twofold in H₂O with 0.1% TFA and reapplied to the column. The column was then developed with a 30 minute linear aqueous acetonitrile gradient (0-60%) containing 0.1% TFA at a flow rate of 0.5 to 1 ml/minute. The fractions obtained were assayed as above. Active fractions were pooled, diluted with H₂O/0.1 % TFA and fractionated under the same conditions to obtain a single protein peak. The fraction corresponding to the peak and flanking fractions were tested in the infectivity assay to verify that suppressor activity was cofractionated with the protein.

Suppressive activity against HIV-1_{IIIB} in the absence of cytotoxic effects consistently copurified with a single protein peak that appeared as a homogeneous 8 kDa band when analyzed by SDS-polyacrylamide gel electrophoresis. This protein was not reactive in ELISAs for RANTES, MIP-1α or MIP-1 β (R&D Systems).

Recombinant gp120 protein derived from HIV-1 IIIB isolate is purchased from Intracel (Foster City, CA).

### 6.1.2 IMMUNIZATION OF MICE

The hMDC and the gp120 is resuspended in a total volume of 50 µl of phosphate-buffered saline (PBS). Mice are divided into 5 groups with 3-4 mice in each group. Groups 1-4 are inoculated with 10 µg gp120 and 0.3 µg, 0.1 µg, 0.03 µg, and 0.01 µg of hMDC, respectively. As a control, group 5 is inoculated with 10 µg of gp120 in the absence of hMDC. For primary inoculation, each group of mice is inoculated with 10 µl of the hMDC and gp120 solution via footpad. Two to three weeks after the primary inoculation, each mouse is given the same does of hMDC/gp120 that is used in primary inoculation.

### 6.1.3 ELISA ASSAY

Serum samples are collected one week after the second inoculation via tail vein bleed. gp120 serum responses are measured using standard gp120 antibody ELISA assays.

### 6.1.4 DTH ASSAY

The delayed-type hypersensitivity (DTH) response is measured from 1-7 days after the second inoculation. Acaliper is to be used to measure footpad swelling.

### 6.2. RESULTS

Mice inoculated with hMDC/gp120 are expected to have greater serum antibody and DTH responses than mice inoculated with gp120 alone. The improved responses will be reflected in either increased titers of serum antibody responses or increased footpad swelling. A dose response effect is expected - increasing the dose of hMDC used is expected to cause a corresponding improvement in the serum and DHT gp120-specific responses.

### 7. EXAMPLE: OTHER CHEMOKINES AND COMBINATIONS OF CHEMOKINES

The foregoing experiments can be repeated using other chemokines. For example, the experiments are preferably repeated using a chemokine selected from the group consisting of: Macrophage-derived chemokine, Monocyte chemotactic protein 1. Regulated upon activation, normal T cell expressed and secreted (and its variants), and B-cell-attracting chemokine 1.

### SEQUENCE LISTING

(1) GENERAL INFORMATION
   (i) APPLICANT: Gallo, Robert C.
      DeVico, Anthony L.
      Garzino, Alfedo
   (ii) TITLE OF THE INVENTION: METHOD AND COMPOSITION TO ENHANCE THE EFFICACY OF A VACCINE USING MACROPHAGE DERIVED CHEMOKINE
   (iii) NUMBER OF SEQUENCES: 2
   (iv) CORRESPONDENCE ADDRESS:
      (A) ADDRESSEE: Pennie & Edmonds LLP
      (B) STREET: 1155 Avenue of the Americas
      (C) CITY: New York
      (D) STATE: New York
      (E) COUNTRY: USA
      (F) ZIP: 10036/2711
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE; FastSEQ Version 2.0
   (vi) CURRENT APPLICATION DATA:
      (A) APPLICATION NUMBER: To be assigned
      (B) FILING DATE: Herewith
      (C) CLASSIFICATION:
   (viii) ATTORNEY/AGENT INFORMATION:
      (A) NAME; Misrock, S. Leslie
      (B) REGISTRATION NUMBER: 18,872
      (C) REFERENCE/DOCKET NUMBER: 8769-029
   (ix) TELECOMMUNICATION INFORMATION:
      (A) TELEPHONE: 212-790-9090
      (B) TELEFAX: 212-869-8864
      (C) TELEX: 66141 PENNIE
(2) INFORMATION FOR SEQ ID NO:1 :
   (i) SEQUENCE CHARACTERISTICS:
      (A) LENGTH: 2923 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: mat_peptide
      (B) LOCATION: 92..298
   (xi) SEQUENCE DESCRIPTION: SEQ ID NO:1: 
GAGACATACA GGACAGAGC ATG GCT CGC CTA CAG ACT GCA CTC CTG GTT GTC 52
Met Ala Arg Leu Gln Thr Ala Leu Val
-24 -20 -15
CTC GTC CTC CTT GCT GTG GCG CTT CAA GCA ACT GAG GCA GGC CCC TAC 100
Leu Val Leu Ala Val Ala Leu Gln Ala Thr Glu Ala Gly Pro Tyr
-10 -5 1
GGC GCC AAC ATG GAA GAC AGC GTC TGC CGT GAT TAC GTC CGT TAC 148
Gly Ala Asn Met Glu Asp Ser Val Cys Arg Asp Tyr Val Arg Tyr
                5                   10                  15
CGT CTG CCC CTG CGC GTG AAA CAC TTC TAC TGG ACC TCA GAC TCC 196
Arg Leu Pro Leu Arg Val Lys His Phe Tyr Trp Thr Ser Asp Ser
20 25 30 35

TGC CCG AGG CCT GGC GTG TTG CTA ACC TTC AGG GAT AAG GAG ATC 244
Cys Pro Arg Pro Gly Val Leu Thr Phe Arg Asp Lys Glu Ile
40 45 50
TGT GCC GAT CCC AGA GTG CCC TGG GTG AAG ATG ATT CTC AAT AAG CTG 292
Cys Ala Asp Pro Arg Val Pro Trp Val Lys Met Ile Leu Asn Lys Leu
55 60 65
AGC CAA TGAAGAGCCT ACTCTGATGA CCGTGGCCTT GGCTCCTCCA GGAAGGCTCA 348
Ser Gln
 
GGAGCCCTAC CTCCCTGCCA TTATAGCTGC TCCCCGCCAG AAGCCTGTGC CAACTCTCTG 408
CATTCCCTGA TCTCCATCCC TGTGGCTGTC ACCCTTGGTC ACCTCCGTGC TGTCACTGCC 468
ATCTCCCCCC TGACCCCTCT AACCCATCCT CTGCCTCCCT CCCTGCAGTC AGAGGGTCCT 528
GTTCCCATCA GCGATTCCCC TGCTTAAACC CTTCCATGAC TCCCCACTGC CCTAAGCTGA 588
GGTCAGTCTC CCAAGCCTGG CATGTGGCCC TCTGGATCTG GGTTCCATCT CTGTCTCCAG 648
CCTGCCCACT TCCCTTCATG AATGTTGGGT TCTAGCTCCC TGTTCTCCAA ACCCATACTA 708
CACATCCCAC TTCTGGGTCT TTGCCTGGGA TGTTGCTGAC ACTCAGAAAG TCCCACCACC 768
TGCACATGTG TAGCCCCACC AGCCCTCCAA GGCATTGCTC GCCCAAGCAG CTGGTAATTC 828
CATTTCATGT ATTAGATGTC CCCTGGCCCT CTGTCCCCTC TTAATAACCC TAGTCACAGT 888
CTCCGCAGAT TCTTGGGATT TGGGGGTTTT CTCCCCCACC TCTCCACTAG TTGGACCAAG 948
GTTTCTAGCT AAGTTACTCT AGTCTCCAAG CCTCTAGCAT AGAGCACTGC AGACAGGCCC 1008
TGGCTCAGAA TCAGAGCCCA GAAAGTGGCT GCAGACAAAA TCAATAAAAC TAATGTCCCT 1068
CCCCTCTCCC TGCCAAAAGG CAGTTACATA TCAATACAGA GACTCAAGGT CACTAGAAAT 1128
GGGCCAGCTG GGTCAATGTG AAGCCCCAAA TTTGCCCAGA TTCACCTTTC TTCCCCCACT 1188
CCCTTTTTTT TTTTTTTTTT TTTGAGATGG AGTTTCGCTC TTGTCACCCA CGCTGGAGTG 1248
CAATGGTGTG GTCTTGGCTT ATTGAAGCCT CTGCCTCCTG GGTTCAAGTG ATTCTCTTGC 1308
CTCAGCCTCC TGAGTAGCTG GGATTACAGG TTCCTGCTAC CACGCCCAGC TAATTTTTGT 1368
ATTTTTAGTA GAGACGAGGC TTCACCATGT TGGCCAGGCT GGTCTCGAAC TCCTGTCCTC 1428
AGGTAATCCG CCCACCTCAG CCTCCCAAAG TGCTGGGATT ACAGGCGTGA GCCACAGTGC 1488
CTGGCCTCTT CCCTCTCCCC ACTGCCCCCC CCAACTTTTT TTTTTTTTTT ATGGCAGGGT 1548
CTCACTCTGT CGCCCAGGCT GGAGTGCAGT GGCGTGATCT CGGCTCACTA CAACCTCGAC 1608
CTCCTGGGTT CAAGTGATTC TCCCACCCCA GCCTCCCAAG TAGCTGGGAT TACAGGTGTG 1668
TGCCACTACG GCTGGCTAAT TTTTGTATTT TTAGTAGAGA CAGGTTTCAC CATATTGGCC 1728
AGGCTGGTCT TGAACTCCTG ACCTCAAGTG ATCCACCTTC CTTGTGCTCC CAAAGTGCTG 1788
AGATTACAGG CGTGAGCTAT CACACCCAGC CTCCCCCTTT TTTTCCTAAT AGGAGACTCC 1848
TGTACCTTTC TTCGTTTTAC CTATGTGTCG TGTCTGCTTA CATTTCCTTC TCCCCTCAGG 1908
CTTTTTTTGG GTGGTCCTCC AACCTCCAAT ACCCAGGCCT GGCCTCTTCA GAGTACCCCC 1968
CATTCCACTT TCCCTGCCTC CTTCCTTAAA TAGCTGACAA TCAAATTCAT GCTATGGTGT 2028
GAAAGACTAC CTTTGACTTG GTATTATAAG CTGGAGTTAT ATATGTATTT GAAAACAGAG 2088
TAAATACTTA AGAGGCCAAA TAGATGAATG GAAGAATTTT AGGAACTGTG AGAGGGGGAC 2148
AAGGTGAAGC TTTCCTGGCC CTGGGAGGAA GCTGGCTGTG GTAGCGTAGC GCTCTCTCTC 2208
TCTGTCTGTG GCAGGAGCCA AAGAGTAGGG TGTAATTGAG TGAAGGAATC CTGGGTAGAG 2268
ACCATTCTCA GGTGGTTGGG CCAGGCTAAA GACTGGGAGT TGGGTCTATC TATGCCTTTC 2328
TGGCTGATTT TTGTAGAGAC GGGGTTTTGC CATGTTACCC AGGCTGGTCT CAAACTCCTG 2388
GGCTCAAGCG ATCCTCCTGG CTCAGCCTCC CAAAGTGCTG GGATTACAGG CGTGAATCAC 2448
TGCGCCTGGC TTCCTCTTCC TCTTGAGAAA TATTCTTTTC ATACAGCAAG TATGGGACAG 2508
CAGTGTCCCA GGTAAAGGAC ATAAATGTTA CAAGTGTCTG GTCCTTTCTG AGGGAGGCTG 2568
GTGCCGCTCT GCAGGGTATT TGAACCTGTG GAATTGGAGG AGGCCATTTC ACTCCCTGAA 2628
CCCAGCCTGA CAAATCACAG TGAGAATGTT CACCTTATAG GCTTGCTGTG GGGCTCAGGT 2688
TGAAAGTGTG GGGAGTGACA CTGCCTAGGC ATCCAGCTCA GTGTCATCCA GGGCCTGTGT 2748
CCCTCCCGAA CCCAGGGTCA ACCTGCCTGC CACAGGCACT AGAAGGACGA ATCTGCCTAC 2808
TGCCCATGAA CGGGGCCCTC AAGCGTCCTG GGATCTCCTT CTCCCTCCTG TCCTGTCCTT 2868
GCCCCTCAGG ACTGCTGGAA AATAAATCCT TTAAAATAGT AAAAAAAAAA AAAAA 2923
(2) INFORMATION FOR SEQ ID NO:2:
   (i) SEQUENCE CHARACTERISTICS:
      (A) LENGTH: 93 amino acids
      (B) TYPE: amino acid
      (D) TOPOLOGY: linear
   (ii) MOLECULE TYPE: protein
   (xi) SEQUENCE DESCRIPTION: SEQ ID NO:2:

Met Ala Arg Leu Gln Thr Ala Leu Val Leu Val Leu Ala
-24 -20 -15 -10
Val Ala Leu Gln Ala Thr Glu Ala Gly Pro Tyr Gly Ala Asn Met Glu
-5 1 5
Asp Ser Val Cys Arg Asp Tyr Val Arg Tyr Arg Leu Pro Leu Arg
10 15 20
Val Lys His Phe Tyr Trp Thr Ser Asp Ser Cys Pro Arg Pro Gly
25 30 35 40
Val Leu Thr Phe Arg Asp Lys Glu Ile Cys Ala Asp Pro Arg
45 50 55
Val Pro Trp Val Lys Met Ile Leu Asn Lys Leu Ser Gln
60 65
C-CHEMOKINES
LYMPHOTACTIN (SCM-1) D63789 D63790

CX3C-chemokines
Fractalkine/neurotactin U91835 U84487

LOCUS       HSU83171     2923 bp    mRNA            PRI       31-MAY-1997
DEFINITION    Human macrophage-derived chemokine precursor (MDC) mRNA,
complete
            cds.
ACCESSION   U83171
NID         g1931580
KEYWORDS    .
SOURCE      human.
  ORGANISM  Homo sapiens
            Eukaryotae; mitochondrial eukaryotes; Metazoa; Chordata;
            Vertebrata;   Mammalia;   Eutheria;   Primates;   Catarrhini;
Hominidae;
            Homo.
REFERENCE   1  (bases 1 to 2923)
  AUTHORS   Godiska,R., Chantry,D., Raport,C.J., Sozzani,S., Allavena,P.,
            Leviten,D., Mantovani,A. and Gray,P.W.
  TITLE         Human macrophage-derived chemokine (MDC), a novel
chemoattractant
            for monocytes, monocyte-derived dendritic cells, and natural
killer
            cells
  JOURNAL   J. Exp. Med. 185 (9), 1595-1604 (1997)
  MEDLINE   97296313
REFERENCE   2  (bases 1 to 2923)
  AUTHORS   Godiska,R. and Gray,P.W.
  TITLE     Direct Submission
  JOURNAL   Submitted (23-DEC-1996) ICOS Corporation, 22021 20th Avenue SE,
            Bothell, WA 98021, USA
FEATURES             Location/Qualifiers
     source          1..2923
                     /organism="Homo sapiens"
                     /db_xref="taxon:9606"
                     /chromosome="16"
     gene            20..301
                     /gene="MDC"
     sig_peptide     20..91
                     /gene="MDC"
     CDS             20..301
                     /gene="MDC"
                     /function="chemotactic for dendritic cells and natural
                     killer cells"
                     /codon_start=1
                     /product="macrophage-derived chemokine precursor"
                     /db_xref="PID:g1931581"

/translation="MARLQTALLVVLVLLAVALQATEAGPYGANMEDSVCCRDYVRYR
                     LPLRVVKHFYWTSDSCPRPGVVLLTFRDKEICADPRVPWVKMILNKLSQ"
     mat_peptide     92..298
                     /gene="MDC"
                     /product="macrophage-derived chemokine"
     repeat_region   complement(1194..1805)
                     /rpt_family="ALU"
     repeat_region   complement(2335..2443)
                     /rpt_family="ALU"
BASE COUNT      605 a    861 c    669 g    788 t
ORIGIN
        1 gagacataca ggacagagca tggctcgcct acagactgca ctcctggttg tcctcgtcct
       61 ccttgctgtg gcgcttcaag caactgaggc aggcccctac ggcgccaaca tggaagacag
      121 cgtctgctgc cgtgattacg tccgttaccg tctgcccctg cgcgtggtga aacacttcta
      181 ctggacctca gactcctgcc cgaggcctgg cgtggtgttg ctaaccttca gggataagga
      241 gatctgtgcc gatcccagag tgccctgggt gaagatgatt ctcaataagc tgagccaatg
      301 aagagcctac tctgatgacc gtggccttgg ctcctccagg aaggctcagg agccctacct
      361 ccctgccatt atagctgctc cccgccagaa gcctgtgcca actctctgca ttccctgatc
      421 tccatccctg tggctgtcac ccttggtcac ctccgtgctg tcactgccat ctcccccctg
      481 acccctctaa cccatcctct gcctccctcc ctgcagtcag agggtcctgt tcccatcagc
      541 gattcccctg cttaaaccct tccatgactc cccactgccc taagctgagg tcagtctccc
      601 aagcctggca tgtggccctc tggatctggg ttccatctct gtctccagcc tgcccacttc
      661 ccttcatgaa tgttgggttc tagctccctg ttctccaaac ccatactaca catcccactt
      721 ctgggtcttt gcctgggatg ttgctgacac tcagaaagtc ccaccacctg cacatgtgta
      781 gccccaccag ccctccaagg cattgctcgc ccaagcagct ggtaattcca tttcatgtat
      841 tagatgtccc ctggccctct gtcccctctt aataacccta gtcacagtct ccgcagattc

901 ttgggatttg ggggttttct cccccacctc tccactagtt ggaccaaggt ttctagctaa
961 gttactctag tctccaagcc tctagcatag agcactgcag acaggccctg gctcagaatc
1021 agagcccaga aagtggctgc agacaaaatc aataaaacta atgtccctcc cctctccctg
1081 ccaaaaggca gttacatatc aatacagaga ctcaaggtca ctagaaatgg gccagctggg
1141 tcaatgtgaa gccccaaatt tgcccagatt cacctttctt cccccactcc cttttttttt
1201 tttttttttt tgagatggag tttcgctctt gtcacccacg ctggagtgca atggtgtggt
1261 cttggcttat tgaagcctct gcctcctggg ttcaagtgat tctcttgcct cagcctcctg
1321 agtagctggg attacaggtt cctgctacca cgcccagcta atttttgtat ttttagtaga
1381 gacgaggctt caccatgttg gccaggctgg tctcgaactc ctgtcctcag gtaatccgcc
1441 cacctcagcc tcccaaagtg ctgggattac aggcgtgagc cacagtgcct ggcctcttcc
1501 ctctccccac tgcccccccc aacttttttt ttttttttat ggcagggtct cactctgtcg
1561 cccaggctgg agtgcagtgg cgtgatctcg gctcactaca acctcgacct cctgggttca
1621 agtgattctc ccaccccagc ctcccaagta gctgggatta caggtgtgtg ccactacggc
1681 tggctaattt ttgtattttt agtagagaca ggtttcacca tattggccag gctggtcttg
1741 aactcctgac ctcaagtgat ccaccttcct tgtgctccca aagtgctgag attacaggcg
1801 tgagctatca cacccagcct cccccttttt ttcctaatag gagactcctg tacctttctt
1861 cgttttacct atgtgtcgtg tctgcttaca tttccttctc ccctcaggct tttttgggt
1921 ggtcctccaa cctccaatac ccaggcctgg cctcttcaga gtacccccca ttccactttc
1981 cctgcctcct tccttaaata gctgacaatc aaattcatgc tatggtgtga aagactacct
2041 ttgacttggt attataagct ggagttatat atgtatttga aaacagagta aatacttaag
2101 aggccaaata gatgaatgga agaattttag gaactgtgag aggggacaa ggtgaagctt
2161 tcctggccct gggaggaagc tggctgtggt agcgtagcgc tctctctctc tgtctgtggc
2221 aggagccaaa gagtagggtg taattgagtg aaggaatcct gggtagagac cattctcagg
2281 tggttgggcc aggctaaaga ctgggagttg ggtctatcta tgcctttctg gctgattttt
2341 gtagagacgg ggttttgcca tgttacccag gctggtctca aactcctggg ctcaagcgat
2401 cctcctggct cagcctccca aagtgctggg attacaggcg tgaatcactg cgcctggctt
2461 cctcttcctc ttgagaaata ttctttttcat acagcaagta tgggacagca gtgtcccagg
2521 taaaggacat aaatgttaca agtgtctggt cctttctgag ggaggctggt gccgctctgc
2581 agggtatttg aacctgtgga attggaggag gccatttcac tccctgaacc cagcctgaca
2641 aatcacagtg agaatgttca ccttataggc ttgctgtggg gctcaggttg aaagtgtggg
2701 gagtgacact gcctaggcat ccagctcagt gtcatccagg gcctgtgtcc ctcccgaacc
2761 cagggtcaac ctgcctgcca caggcactag aaggacgaat ctgcctactg cccatgaacg
2821 gggccctcaa gcgtcctggg atctccttct ccctcctgtc ctgtccttgc ccctcaggac
2881 tgctggaaaa taaatccttt aaaatagtaa aaaaaaaaaa aaa

LOCUS       HSMCP1        725 bp    RNA             PRI       03-APR-1995
DEFINITION  H.sapiens mRNA for monocyte chemoattractant protein 1 (MCP-1).
ACCESSION   X14768
NID         g34513
KEYWORDS    monocyte chemoattractant protein 1.
SOURCE      human.
  ORGANISM  Homo sapiens
            Eukaryotae; mitochondrial eukaryotes; Metazoa; Chordata;
            Vertebrata; Eutheria; Primates; Catarrhini; Hominidae; Homo.
REFERENCE   1  (bases 1 to 725)
  AUTHORS   Yoshimura,T., Yuhki,N., Moore,S.K., Appella,E., Lerman,M.I. and
            Leonard,E.J.
  TITLE      Human monocyte chemoattractant protein-1 (MCP-1). Full-length
cDNA
            cloning, expression in mitogen-stimulated blood mononuclear
            leukocytes, and sequence similarity to mouse competence gene JE
  JOURNAL   FEBS Lett. 244 (2), 487-493 (1989)
  MEDLINE   89153605
COMMENT     ZAPII.
FEATURES             Location/Qualifiers
     source          1..725
                     /organism="Homo sapiens"
                     /db_xref="taxon:9606"
                     /cell_type="glioma cells"
                     /cell_line="U105MG"
                     /clone_lib="lambda"
     sig_peptide     54..122
                     /note="signal peptide (AA -23 to -1)"
     CDS             54..353
                     /codon_start=1
                     /product="monocyte chemoattractant preprotein"
                     /db_xref="PID:g34514"
                     /db_xref="SWISS-PROT:P13500"

/translation="MKVSAALLCLLLIAATFIPQGLAQPDAINAPVTCCYNFTNRKIS

VQRLASYRRITSSKCPKEAVIFKTIVAKEICADPKQKWVQDSMDHLDKQTQTPKT"
     mat_peptide     123..350
                     /note="MCP-1 (AA 1 - 76)"
     misc_feature    162..170
                     /note="pot. N-linked glycosylation site"
     misc_feature    707..712
                     /note="pot. polyA signal"
     polyA_site      725
                     /note="polyA site"
BASE COUNT      208 a    171 c    126 g    220 t
ORIGIN
        1 ctaacccaga aacatccaat tctcaaactg aagctcgcac tctcgcctcc agcatgaaag
       61 tctctgccgc ccttctgtgc ctgctgctca tagcagccac cttcattccc caagggctcg
      121 ctcagccaga tgcaatcaat gccccagtca cctgctgtta taacttcacc aataggaaga
      181 tctcagtgca gaggctcgcg agctatagaa gaatcaccag cagcaagtgt cccaaagaag

      241 ctgtgatctt caagaccatt gtggccaagg agatctgtgc tgaccccaag cagaagtggg
      301 ttcaggattc catggaccac ctggacaagc aaacccaaac tccgaagact tgaacactca
      361 ctccacaacc caagaatctg cagctaactt attttcccct agctttcccc agacaccctg
      421 ttttatttta ttataatgaa ttttgtttgt tgatgtgaaa cattatgcct taagtaatgt
      481 taattcttat ttaagttatt gatgttttaa gtttatcttt catggtacta gtgtttttta
      541 gatacagaga cttggggaaa ttgcttttcc tcttgaacca cagttctacc cctgggatgt
      601 tttgagggtc tttgcaagaa tcattaatac aaagaatttt ttttaacatt ccaatgcatt
      661 gctaaaatat tattgtggaa atgaatattt tgtaactatt acaccaaata aatatatttt
      721 tgtac

//
LOCUS       HUMTCSM      1160 bp    mRNA            PRI       15-JUN-1989
DEFINITION  Human T cell-specific protein (RANTES) mRNA, complete cds.
ACCESSION   M21121
NID         g339420

KEYWORDS    Alu repeat; T-cell-specific protein.
SOURCE      Human peripheral blood (T lymphocyte) cell line AH2, cDNA to
mRNA,
            clone 228.
  ORGANISM  Homo sapiens
            Eukaryotae; mitochondrial eukaryotes; Metazoa; Chordata;
            Vertebrata; Eutheria; Primates; Catarrhini; Hominidae; Homo.
REFERENCE   1  (bases 1 to 1160)
  AUTHORS   Schall,T.J.,   Jongstra,J.,   Dyer,B.J.,   Jorgensen,J.,
Clayberger,C.,
            Davis,M.M. and Krensky,A.M.
  TITLE     A human T cell-specific molecule is a member of a new gene
family
  JOURNAL   J. Immunol. 141, 1018-1025 (1988)
  MEDLINE   88285659
COMMENT     Draft entry and computer-readable sequence for [1] kindly
provided
            by A.M.Krensky, 24-OCT-1988.
FEATURES             Location/Qualifiers
     source          1..1160
                     /organism="Homo sapiens"
                     /db_xref="taxon:9606"
     CDS             27..302
                     /note="T cell-specific protein precursor"
                     /codon_start=1
                     /db_xref="PID:g339421"

/translation="MKVSAARLAVILIATALCAPASASPYSSDTTPCCFAYIARPLPR
                     AHIKEYFYTSGKCSNPAVVFVTRKNRQVCANPEKKWVREYINSLEMS"
     sig_peptide     27..95
                     /note="T cell-specific protein signal peptide"
     mat_peptide     96..299
                     /note="T cell-specific protein"
     repeat_region   450..950
                     /note="Alu-related repeats"
BASE COUNT      298 a    332 c    295 g    235 t
ORIGIN      276 bp upstream of RsaI site.
        1 cctccgacag cctctccaca ggtaccatga aggtctccgc ggcacgcctc gctgtcatcc
       61 tcattgctac tgccctctgc gctcctgcat ctgcctcccc atattcctcg gacaccacac
      121 cctgctgctt tgcctacatt gcccgcccac tgccccgtgc ccacatcaag gagtatttct
      181 acaccagtgg caagtgctcc aacccagcag tcgtctttgt cacccgaaag aaccgccaag
      241 tgtgtgccaa cccagagaag aaatgggttc gggagtacat caactctttg gagatgagct
      301 aggatggaga gtccttgaac ctgaacttac acaaatttgc ctgtttctgc ttgctcttgt
      361 cctagcttgg gaggcttccc ctcactatcc taccccaccc gctccttgaa gggcccagat
      421 tctgaccacg acgagcagca gttacaaaaa ccttccccag gctggacgtg gtggctcagc
      481 cttgtaatcc cagcactttg ggaggccaag gtgggtggat cacttgaggt caggagttcg
      541 agacagcctg gccaacatga tgaaacccca tgtgtactaa aaatacaaaa aattagccgg
      601 gcgtggtagc gggcgcctgt agtcccagct actcgggagg ctgaggcagg agaatggcgt
      661 gaacccggga gcggagcttg cagtgagccg agatcgcgcc actgcactcc agcctgggcg
      721 acagagcgag actccgtctc aaaaaaaaaa aaaaaaaaaa aaaaaataca aaaattagcc
      781 gcgtggtggc ccacgcctgt aatcccagct actcgggagg ctaaggcagg aaaattgttt
      841 gaacccagga ggtggaggct gcagtgagct gagattgtgc cacttcactc cagcctgggt
      901 gacaaagtga gactccgtca caacaacaac aacaaaaagc ttccccaact aaagcctaga
      961 agagcttctg aggcgctgct ttgtcaaaag gaagtctcta ggttctgagc tctggctttg
     1021 ccttggcttt gcaagggctc tgtgacaagg aaggaagtca gcatgcctct agaggcaagg
     1081 aagggaggaa cactgcactc ttaagcttcc gccgtctcaa cccctcacag gagcttactg
     1141 gcaaacatga aaaatcgggg
//

LOCUS       HSJ002211     663 bp    mRNA            PRI       11-MAR-1998
DEFINITION  Homo sapiens cDNA for a CXC chemokine.
ACCESSION   AJ002211

NID         g2832410
KEYWORDS    CXC chemokine.
SOURCE      human.
  ORGANISM  Homo sapiens
            Eukaryota; Metazoa; Chordata; Vertebrata; Mammalia; Eutheria;
            Primates; Catarrhini; Hominidae; Homo.
REFERENCE   1  (bases 1 to 663)
  AUTHORS      Legler,D.F.,   Loetscher,M.,   Roos,R.S.,   Clark-Lewis,I.,
Baggiolini,M.
            and Moser,B.
  TITLE     B cell-attracting chemokine 1, a human CXC chemokine expressed
in
            lymphoid  tissues,  selectively  attracts  B  lymphocytes  via
BLR1/CXCR5
  JOURNAL   J. Exp. Med. 187 (4), 655-660 (1998)
  MEDLINE   98130629
REFERENCE   2  (bases 1 to 663)
  AUTHORS   Moser,B.
  TITLE     Direct Submission
  JOURNAL   Submitted (05-NOV-1997) Moser B., University of Bern, Theodor
            Kocher Institute, Freiestrasse 1, CH-3012 Bern, SWITZERLAND
FEATURES             Location/Qualifiers
     source          1..663
                     /organism="Homo sapiens"
                     /db_xref="taxon:9606"
                     /cell_type="PBL"
     sig_peptide     35..100
                     /gene="BCA-1"
     CDS             35..364
                     /gene="BCA-1"
                     /codon_start=1
                     /product="CXC chemokine"
                     /db_xref="PID:e1249325"
                     /db_xref="PID:g2832411"

/translation="MKFISTSLLLMLLVSSLSPVQGVLEVYYTSLRCRCVQESSVFIP

RRFIDRIQILPRGNGCPRKSIIVWKKNKSIVCVDPQAEWIQRMMEVLRKRSSSTLPVP
                     VFKRKIP"
     gene            35..364
                     /gene="BCA-1"
     mat_peptide     101..361
                     /gene="BCA-1"
BASE COUNT      176 a    136 c    145 g    198 t     8 others
ORIGIN
        1 cagagctcaa gtctgaactc tacctccaga cagaatgaag ttcatctcga catctctgct
       61 tctcatgctg ctggtcagca gcctctctcc agtccaaggt gttctggagg tctattacac
      121 aagcttgagg tgtagatgtg tccaagagag ctcagtcttt atccctagac gcttcattga
      181 tcgaattcaa atcttgcccc gtgggaatgg ttgtccaaga aaagaaatca tagtctggaa
      241 gaagaacaag tcaattgtgt gtgtggaccc tcaagctgaa tggatacaaa gaatgatgga
      301 agtattgaga aaaagaagtt cttcaactct accagttcca gtgtttaaga gaaagattcc
      361 ctgatgctga tatttccact aagaacacct gcattcttcc cttatccctg ctctgggatt
      421 ttagttttgt gcttagttaa atcttttcca gggagaaaga acttccccat acaaataagg
      481 catgaggact atgtaaaaat aaccttgcag gagctggatg gggggccaaa ctcaagcttc
      541 tttcactcca caggcaccct attntacact tgggggtttt gcnttctttn tttcntcagg
      601 gggggggaaa gtttcttttg gaaantagtt nttccagttn ttaggtatta cagggttntt
      661 ttt

## Claims

1. A composition comprising: an immunogenic amount of a purified gp120 or gp160 HIV glycoprotein antigen and an amount of a purified chemokine selected from the group consisting of MDC, BLC, RANTES and MCP-1, or purified fragments thereof, effective to enhance the immune response to said antigen; and a pharmaceutically acceptable carrier.

2. The composition of claim 1, wherein the fragment(s) are truncation isoforms.

3. The composition of claim 1, wherein the chemokine fragment includes an MDC fragment selected from the group consisting of amino acid numbers 2-69, 3-69, 5-69, 7-69 and 9-69 of SEQ ID NO: 2.

4. The composition of claim 1, wherein the chemokine is a human chemokine.

5. The composition of claim 1, wherein the antigen is HIV associated gp120 protein.

6. A composition comprising an amount of a first set of purified nucleic acids comprising a, nucleotide sequence encoding a gp120 or gp160 HIV glycoprotein antigen and a second set of purified nucleic acids comprising a nucleotide sequence encoding a chemokine selected from the group consisting of MDC, BLC, RANTES and Mop-1, or fragments thereof, wherein the antigen and the chemokine, or fragment(s) thereof, are expressed from said first set of nucleic acids and second set of nucleic acids in a coordinated manner such that upon introduction into a suitable cell, the amount of said first set of nucleic acids is sufficient to express an immunogenic amount of the antigen and the amount of the said second set of nucleic acids is sufficient to express an amount of chemokine that is effective in enhancing the immune response to the HIV antigen; and a pharmaceutically acceptable carrier.

7. The composition of claim 6, wherein the chemokine is MDC and the nucleic acid encoding the MDC comprises the nucleotide sequence of SEQ ID NO: 1.

8. The composition of claim 6, further comprising pharmaceutically acceptable excipient, auxiliary substance, adjuvant, wetting or emulsifying agent, or pH buffering agent.

9. The composition of claim 6, wherein the nucleic acid is introduced into a suitable host cell.

10. A composition according to any preceding claim for enhancing the immune response to HIV in a subject.

11. Use of the composition of any preceding claim for the manufacture of a medicament for enhancing the immune response to HIV in a subject.

## Patentansprüche

1. Zusammensetzung, die Folgendes umfasst: eine immunogene Menge eines gereinigten gp120 oder pg160 HIV-Glycoprotein-Antigens und eine Menge eines gereinigten Chemokins, ausgewählt aus der Gruppe bestehend aus MDC, BLC, RANTES und MCP-1 oder gereinigten Fragmenten davon, mit der Wirkung, die Immunantwort auf das genannte Antigen zu verstärken; und einen pharmazeutisch verträglichen Träger.

2. Zusammensetzung nach Anspruch 1, wobei das/die Fragment(e) (eine) Trunkierungsisoform(en) ist/sind.

3. Zusammensetzung nach Anspruch 1, wobei das Chemokinfragment ein MDC-Fragment enthält, das ausgewählt ist aus der Gruppe bestehend aus Aminosäurenummern 2-69, 3-69, 5-69, 7-69 und 9-69 von SEQ ID NO: 2.

4. Zusammensetzung nach Anspruch 1, wobei das Chemokin ein humanes Chemokin ist.

5. Zusammensetzung nach Anspruch 1, wobei das Antigen ein HIV-assoziiertes gp120 Protein ist.

6. Zusammensetzung, die eine Menge eines ersten Satzes von gereinigten Nukleinsäuren, mit einer gp120 oder gp160 HIV-Glycoprotein-Antigen codierenden Nukleotidsequenz, und einen zweiten Satz von gereinigten Nukleinsäuren umfasst, mit einer Nukleotidsequzenz, die ein Chemokin codiert, das ausgewählt ist aus der Gruppe bestehend aus MDC, BLC, RANTES und MCP-1 oder Fragmenten davon, wobei das Antigen und das Chemokin, oder (ein) Fragment(e) davon, von dem genannten ersten Satz von Nukleinsäuren und dem genannten zweiten Satz von Nukleinsäuren auf eine koordinierte Weise exprimiert werden, so dass nach Einleitung in eine geeignete Zelle die Menge an dem genannten ersten Satz von Nukleinsäuren ausreicht, um eine immunogene Menge des Antigens zu exprimieren, und die Menge des genannten zweiten Satzes von Nukleinsäuren ausreicht, um eine Menge an Chemokin zu exprimieren, die die Immunantwort auf das HIV-Antigen wirksam verstärken kann; und einen pharmazeutisch verträglichen Träger.

7. Zusammensetzung nach Anspruch 6, wobei das Chemokin MDC ist und die das MDC codierende Nukleinsäure die Nukleotidsequenz von SEQ ID NO: 1 umfasst.

8. Zusammensetzung nach Anspruch 6, die ferner ein/e/n pharmazeutisch verträglichen Träger, Zusatzsubstanz, Adjuvans, Benetzungs- oder Emulgiermittel oder pH-Puffermittel umfasst.

9. Zusammensetzung nach Anspruch 6, wobei die Nukleinsäure in eine geeignete Wirtszelle eingeführt wird.

10. Zusammensetzung nach einem der vorherigen Ansprüche zum Verbessern der Immunantwort auf HIV in einer Person.

11. Verwendung der Zusammensetzung nach einem der vorherigen Ansprüche für die Herstellung eines Medikaments zum Verstärken der Immunantwort auf HIV in einer Person.

## Revendications

1. Composition comprenant : une quantité immunogène d'un antigène de glycoprotéine du VIH gp120 ou gp160 purifié et une quantité d'une chimiokine purifiée sélectionnée parmi le groupe constitué de MDC, BLC, RANTES et MCP-1, ou de fragments purifiés de celle-ci, efficaces pour amplifier la réponse immunitaire audit antigène ; et un support pharmaceutiquement acceptable.

2. Composition selon la revendication 1, dans laquelle le(s) fragment(s) sont des isoformes de troncature.

3. Composition selon la revendication 1, dans laquelle le fragment de chimiokine comprend un fragment de MDC sélectionné parmi le groupe constitué des acides aminés numéros 2 à 69, 3 à 69, 5 à 69, 7 à 69 et 9 à 69 de SEQ ID N° :2.

4. Composition selon la revendication 1, dans laquelle la chimiokine est une chimiokine humaine.

5. Composition selon la revendication 1, dans laquelle l'antigène est la protéine gp120 associée au VIH.

6. Composition comprenant une quantité d'un premier ensemble d'acides nucléiques purifiés comprenant une séquence nucléotidique codant pour un antigène de glycoprotéine du VIH gp120 ou gp160 et un second ensemble d'acides nucléiques purifiés comprenant une séquence nucléotidique codant pour une chimiokine sélectionnée parmi le groupe constitué de MDC, BLC, RANTES et MCP-1, ou de fragments de celle-ci, dans laquelle l'antigène et la chimiokine, ou un/des fragment(s) de celle-ci, sont exprimés à partir desdits premier ensemble d'acides nucléiques et second ensemble d'acides nucléiques d'une manière coordonnée de sorte que, lors de l'introduction dans une cellule appropriée, la quantité dudit premier ensemble d'acides nucléiques est suffisante pour exprimer une quantité immunogène de l'antigène et la quantité dudit second ensemble d'acides nucléiques est suffisante pour exprimer une quantité de chimiokine qui est efficace pour amplifier la réponse immunitaire à l'antigène du VIH ; et un support pharmaceutiquement acceptable.

7. Composition selon la revendication 6, dans laquelle la chimiokine est MDC et l'acide nucléique codant pour la MDC comprend la séquence nucléotidique de SEQ ID N° : 1.

8. Composition selon la revendication 6, comprenant en outre un excipient pharmaceutiquement acceptable, une substance auxiliaire, un adjuvant, un agent mouillant ou émulsifiant, ou un agent tamponnant le pH.

9. Composition selon la revendication 6, dans laquelle l'acide nucléique est introduit dans une cellule hôte appropriée.

10. Composition selon l'une quelconque des revendications précédentes pour amplifier la réponse immunitaire au VIH chez un sujet.

11. Utilisation de la composition selon l'une quelconque des revendications précédentes pour la fabrication d'un médicament pour amplifier la réponse immunitaire au VIH chez un sujet.
